(19) **Europäisches Patentamt / European Patent Office / Office européen des brevets**

(11) **EP 1 669 030 B1**

(12) **EUROPEAN PATENT SPECIFICATION**

(45) Date of publication and mention
of the grant of the patent:
**24.11.2010 Bulletin 2010/47**

(51) Int Cl.:
*A61B 8/12* (2006.01)       *A61B 5/06* (2006.01)
*A61B 8/14* (2006.01)

(21) Application number: **06005792.4**

(22) Date of filing: **11.06.2003**

(54) **Ultrasonic diagnosis apparatus**

Ultraschalldiagnosegerät

Appareil de diagnostic par ultrasons

(84) Designated Contracting States:
**DE FR GB**

(43) Date of publication of application:
**14.06.2006 Bulletin 2006/24**

(62) Document number(s) of the earlier application(s) in
accordance with Art. 76 EPC:
**03012282.4 / 1 491 146**

(73) Proprietor: **Olympus Corporation**
**Tokyo 151-0072 (JP)**

(72) Inventors:
• **Kawashima, Tomonao**
**c/o Olympus Optical Co., Ltd.**
**Hachioji-shi**
**Tokyo (JP)**

• **Sabata, Tomohiro**
**Hachioji-shi**
**Tokyo (JP)**

(74) Representative: **von Hellfeld, Axel**
**Wuesthoff & Wuesthoff**
**Patent- und Rechtsanwälte**
**Schweigerstrasse 2**
**81541 München (DE)**

(56) References cited:
WO-A-98/29032       WO-A-02/064011
US-A- 5 081 993       US-A- 5 876 345
US-B1- 6 248 074

EP 1 669 030 B1

**Description**

BACKGROUND OF THE INVENTION

1. Field of the Invention

The present invention relates to an ultrasonic diagnosis apparatus and, in particular, to an ultrasonic diagnosis apparatus for performing three-dimensional image processing.

2. Description of the Related Art

The observation of the position and/or spread of a tumor in body tissue, the travelling and/or the form of the blood, the degree of infiltration of the tumor into the blood vessel, and/or the spread of the tumor along the bloodstream are medically important for diagnosing the degree of progress of the disease and/or the spread, for determining the operation method and/or for performing prognoses for patients. In this case, the diagnosis based on three-dimensional images of tissue containing a tumor and/or the bloodstream and tissue containing the bloodstream or the diagnosis based on ultrasonic tomographic images in multiple directions are clinically effective. In the extracorporeal ultrasonic diagnosis field for externally irradiating ultrasound, related technologies as disclosed in Japanese Unexamined Patent Application Publication No. 6-254097 and Japanese Unexamined Patent Application Publication No. 2000-242766 have been known.

On the other hand, recently, an in-body-cavity ultrasonic diagnosis technology has been proposed for irradiating ultrasound to a target organ in the body cavity from the tube cavity in the body cavity having less influence of the attenuation, for example due to fat, by using various kinds of ultrasonic probe such as an ultrasonic endoscope. In the field too, the diagnosis based on three-dimensional images of tissue containing a tumor and/or the bloodstream and tissue containing the bloodstream or the diagnosis based on ultrasonic tomographic image in multiple directions are known as being effective in order to diagnose the infiltration of the tumor in the digestive tract, observe an esophagus varix and observe the bloodstream around a tumor in the digestive tract. The related technologies such as those disclosed in Japanese Unexamined Patent Application Publication No. 2001-161693, Japanese Unexamined Patent Application Publication No. 6-261900 and Japanese Unexamined Patent Application Publication No. 11-113913 have been proposed.

The ultrasonic diagnosis apparatus disclosed in Japanese Unexamined Patent Application Publication No. 2001-161693 performs spiral scanning combining mechanical radial scanning and mechanical linear scanning. By performing the spiral scanning, the ultrasonic diagnosis apparatus can obtain data for displaying ultrasonic three-dimensional images for performing a dual plane reconstruction (DPR) display, and for displaying simultaneously a radial image and a linear image having different observing directions and for displaying ultrasonic three-dimensional images.

The ultrasonic diagnosis apparatus disclosed in Japanese Unexamined Patent Application Publication No. 6-261900 and Japanese Unexamined Patent Application Publication No. 11-113913 has the combination of an ultrasonic probe (including an ultrasonic endoscope) for performing mechanical radial scanning and a position detector. The ultrasonic diagnosis apparatus constructs an ultrasonic three-dimensional image by obtaining data for displaying the three-dimensional image based on information from the position detector, relating to the position and orientation of an ultrasonic scanning plane.

Furthermore, an ultrasonic diagnosis apparatus has been conventionally known for obtaining ultrasonic three-dimensional image data by using an ultrasonic endoscope for obtaining a tomographic image parallel to the inserting axis by using an ultrasonic transducer array along the inserting axis and by rotating the endoscope about the inserting axis. The endoscope may be an ultrasonic endoscope, such as an electronic linear scanning type or electronic convex scanning type ultrasonic endoscope for obtaining Doppler data.

Document US 5,876,345 concerns an ultrasonic catheter having at least two ultrasonic arrays, whereby one ultrasonic array is used as an imaging array and another ultrasonic array is used as a tracking array.

Document US 6,248,074 B1 concerns an ultrasonic diagnosis system. In one embodiment, a magnetic field source is attached to a distal part of a position detection catheter and a magnetic sensor is installed externally.

SUMMARY OF THE INVENTION

The present invention concerns an ultrasonic diagnosis apparatus having the features of claim 1.

An ultrasonic diagnosis apparatus according to the invention for obtaining ultrasonic echo signals of a part to be examined from a probe in a body cavity, includes an ultrasonic probe, a detector and a voxel data creating circuit. The ultrasonic probe has at predetermined positions of an inserting axis a plurality of ultrasonic transducers on the circumference about the inserting axis for performing ultrasonic scanning by using the plurality of ultrasonic transducers on a plane perpendicular to the inserting axis by sending and receiving ultrasound to and from the plurality by ultrasonic transducers. The detector detects the position or orientation of the scanned plane by the ultrasonic scanning by the ultrasonic probe. The voxel data creating circuit creates voxel data based on ultrasonic tomographic image data from echo signals serially obtained by the ultrasonic scanning by the ultrasonic probe and based on the position or orientation data detected by the detector.

BRIEF DESCRIPTION OF THE DRAWINGS

Fig. 1 is a diagram for explaining the entire construction of an ultrasonic diagnosis apparatus according to a first embodiment of the invention;
Fig. 2 is a diagram for explaining the construction of an electronic radial scanning type ultrasonic endoscope according to the first embodiment;
Fig. 3 is a block diagram of a sending/receiving circuit according to the first embodiment;
Fig. 4 is a flowchart showing an example of a processing flow for data recording according to the first embodiment;
Fig. 5 is a flowchart showing an example of a processing flow for three-dimensional image creation according to the first embodiment;
Fig. 6 is a diagram for explaining an operation of a cube data creating circuit according to the first embodiment;
Fig. 7 is a diagram showing an example of monitor screen display according to the first embodiment;
Fig. 8 is a diagram for explaining a relationship of coordinate systems according to the first embodiment;
Fig. 9 is a diagram showing a variation example of the monitor screen display example according to the first embodiment;
Fig. 10 is a diagram for explaining the entire construction of an ultrasonic diagnosis apparatus according to a second embodiment of the invention;
Fig. 11 is a flowchart showing an operation flow of the ultrasonic diagnosis apparatus according to the second embodiment;
Fig. 12 is a diagram showing a monitor screen example according to the second embodiment;
Fig. 13 is a diagram for explaining a variation example of the entire construction according to the second embodiment;
Fig. 14 is a diagram showing a variation example of the monitor screen according to the second embodiment;
Fig. 15 is a diagram for explaining the entire construction of an ultrasonic diagnosis apparatus according to a third embodiment of the invention;
Fig. 16 is a flowchart showing an operation flow of the ultrasonic diagnosis apparatus according to the third embodiment;
Fig. 17 is a diagram showing a monitor screen display example according to the third embodiment;
Fig. 18 is a diagram for explaining the entire construction of an ultrasonic diagnosis apparatus according to a fourth embodiment of the invention;
Fig. 19 is a flowchart showing an operation flow of an ultrasonic diagnosis apparatus according to the fourth embodiment; and
Fig. 20 is a diagram showing a monitor screen display example according to the fourth embodiment.

DETAILED DESCRIPTION OF PREFERRED EMBODIMENTS

Embodiments of the present invention will be described below with reference to drawings.

[First Embodiment]

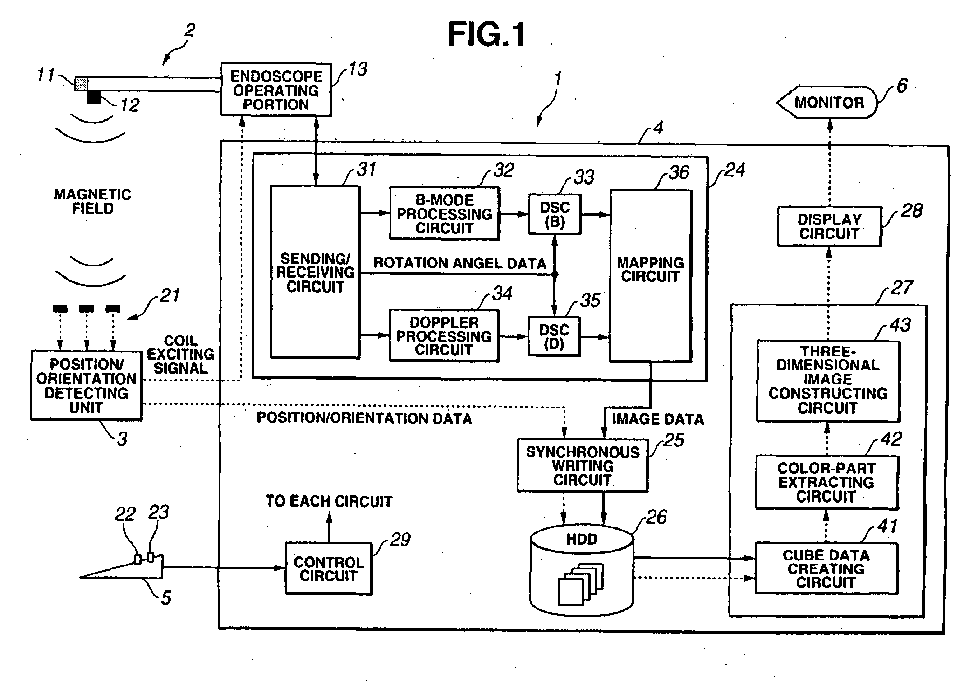

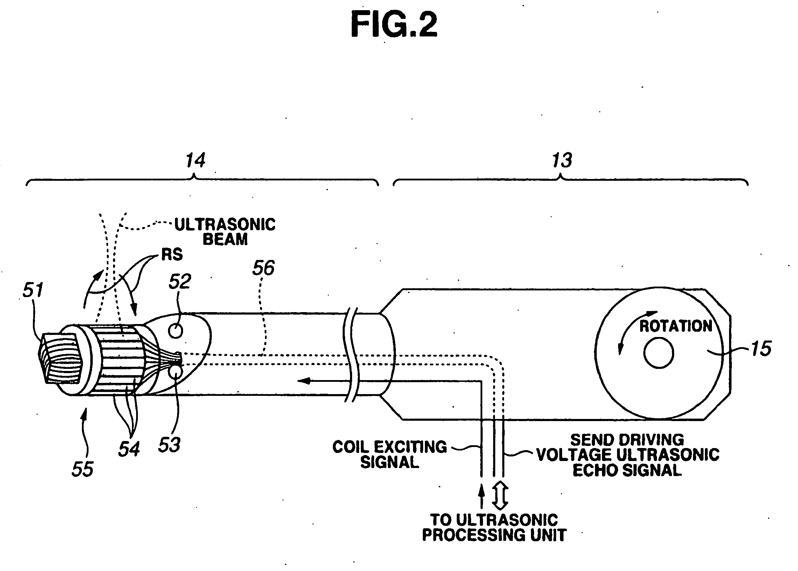

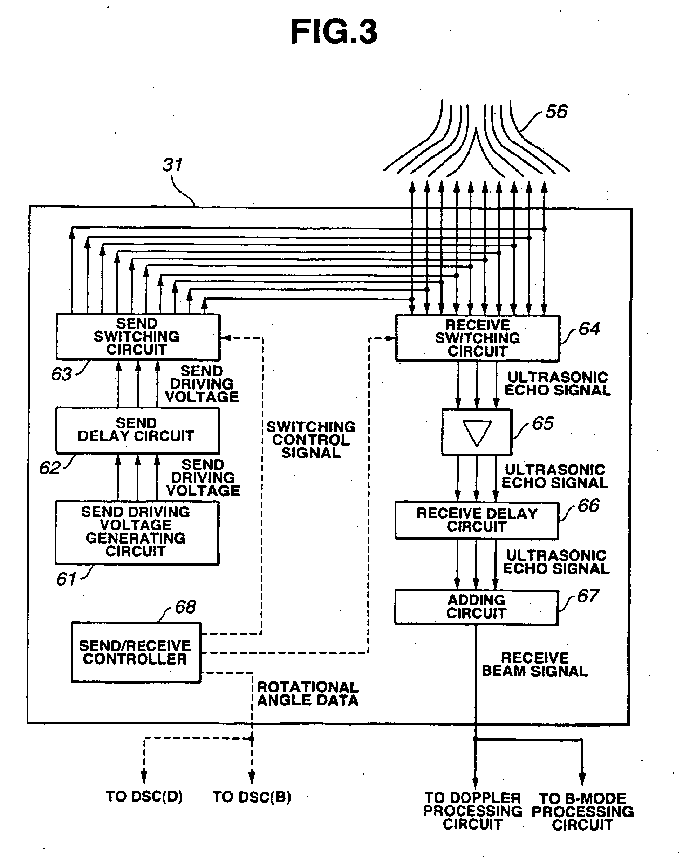

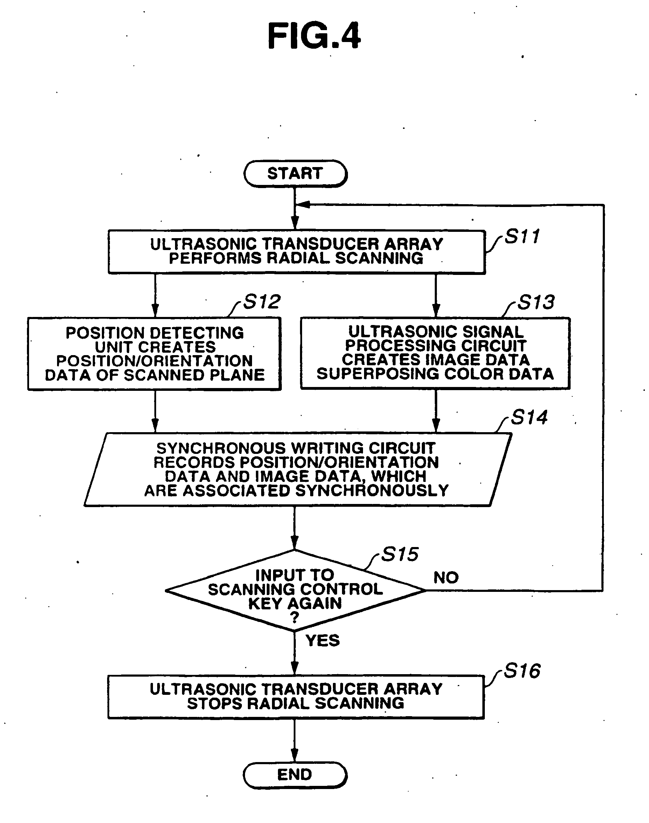

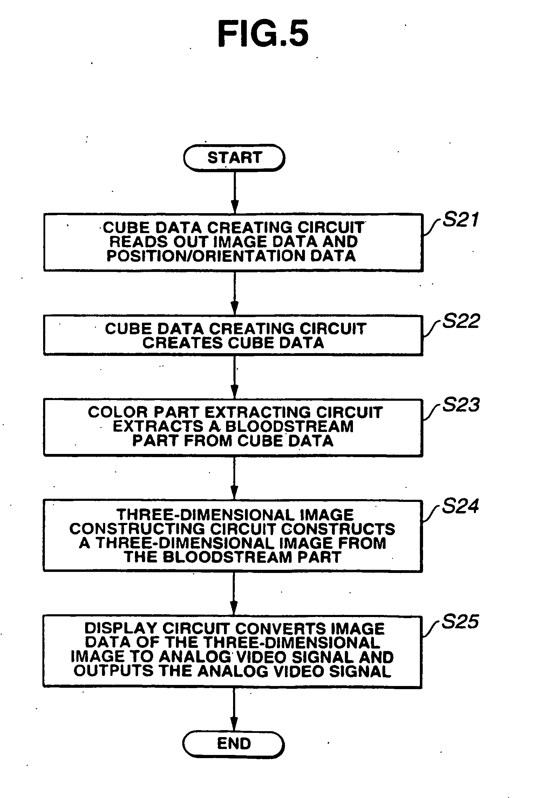

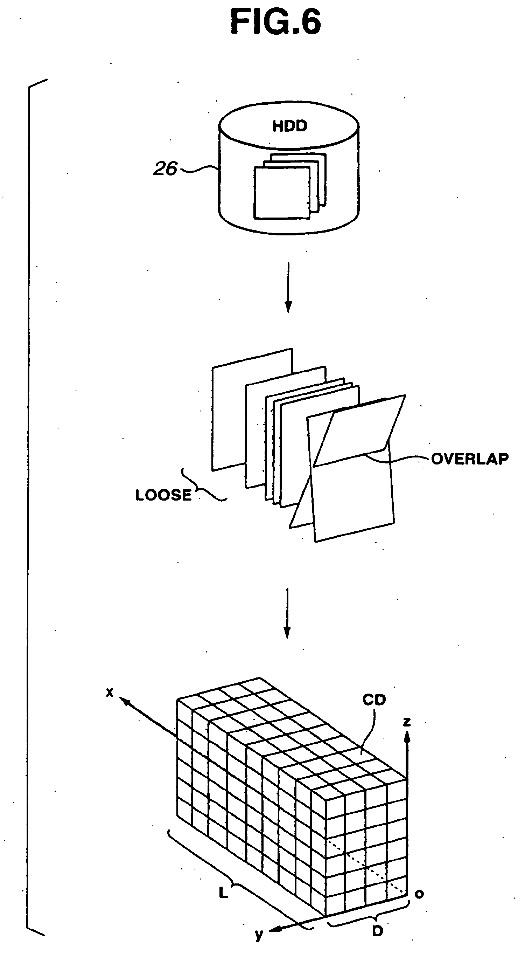

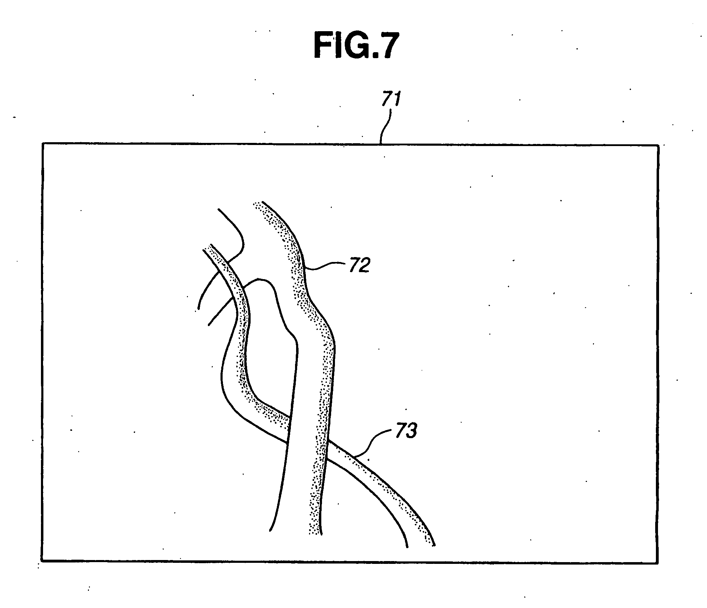

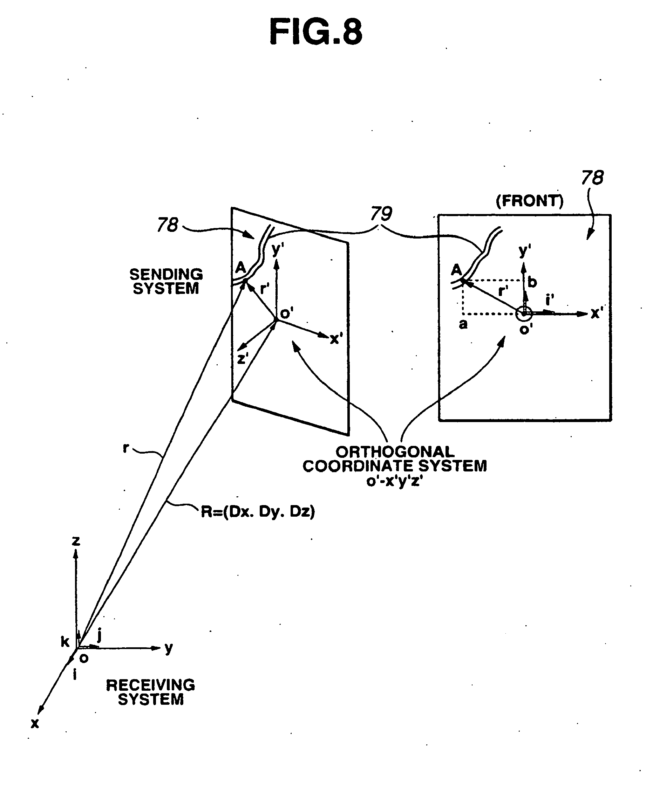

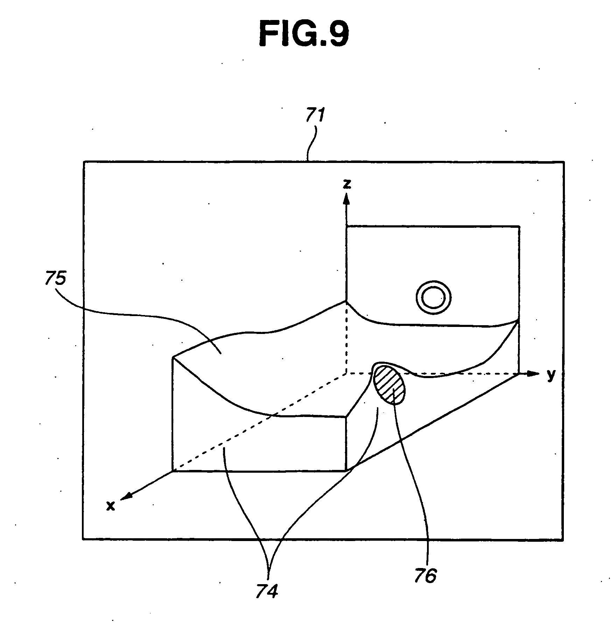

Figs. 1 to 9 are diagrams showing a first embodiment of the invention. Fig. 1 is a diagram for explaining the entire construction of an ultrasonic diagnosis apparatus according to the first embodiment. Fig. 2 is a diagram for explaining the construction of an electronic radial scanning type ultrasonic endoscope according to the first embodiment. Fig. 3 is a block diagram of a sending/receiving circuit according to the first embodiment. Fig. 4 is a flowchart showing an example of a processing flow for data recording according to the first embodiment. Fig. 5 is a flowchart showing an example of a processing flow for three-dimensional image creation according to the first embodiment. Fig. 6 is a diagram for explaining an operation of a cube data creating circuit according to the first embodiment. Fig. 7 is a diagram showing an example of monitor screen display according to the first embodiment. Fig. 8 is a diagram for explaining a relationship of coordinate systems according to the first embodiment. Fig. 9 is a diagram showing a variation example of the monitor screen display example according to the first embodiment.
First of all the entire construction will be described with reference to Fig. 1.
As shown in Fig. 1, an ultrasonic diagnosis apparatus 1 according to this embodiment has an electronic radial scanning type ultrasonic endoscope 2, a position/orientation detecting portion 3, an ultrasonic processing portion 4, a keyboard 5 and a monitor 6.

In Fig. 1, the thick dotted line arrow indicates a signal or data flow relating to a three-dimensional image. The thick solid line arrow indicates a signal or data flow relating to an original image. A thin dotted line arrow indicates signal or data flow relating to a position and/or a direction. A thin solid line arrow indicates the other signal, such as a control signal, or data flow.

The electronic radial scanning type ultrasonic endoscope 2 has at the distal end a ring-shaped ultrasonic transducer array 11 and a sending coil 12 for sending a magnetic field. An operating portion 13 is operated such that an ultrasonic beam is rotated on a section perpendicular to an inserting axis for performing scanning, that is, for performing so-called electronic radial scanning. The position/orientation detecting portion 3 includes multiple receiving coils 21 being spatially fixed and having different orientations. Based on current output by the receiving coils 21 for detecting a magnetic field, the position and orientation of the scanned plane by the electronic radial scanning type ultrasonic endoscope 2 are detected remotely. The ultrasonic processing portion 4 processes ultrasonic echo signals from the ultrasonic endoscope 2 and position and orientation data from the position/orientation detecting portion 3 and constructs a desired three-dimensional image. The keyboard 5 includes a scanning control key 22 and a three-dimensional key (called 3D key, hereinafter) 23, which will be described later. The keyboard 5 is used for externally controlling the ultrasonic processing portion 4. The monitor 6 displays three-dimensional images.

The ultrasonic processing portion 4 includes an ultrasonic signal processing circuit 24, a synchronous writing circuit 25, a hard disk (called "HDD" hereinafter) 26, an image processing circuit 27, a display circuit 28 and a control circuit 29.

The ultrasonic signal processing circuit 24 creates image data of an ultrasonic tomographic image based on ultrasonic echo signals. The synchronous writing circuit 25 controls the writing of image data and position and orientation data to the HDD by synchronizing and associating the image data and the position and orientation data. The image processing circuit 27 constructs three-dimensional images. The display circuit 28 converts image data of the constructed three-dimensional image to analog video signals and outputs so as to be displayed on the monitor 6. The control circuit 29 outputs different kinds of control signals for controlling the respective circuits within the ultrasonic processing portion 4.

The ultrasonic signal processing circuit 24 includes a sending/receiving circuit 31, a B-mode processing circuit 32, a digital scan converter (simply called "DSC(B)" hereinafter) 33, a Doppler processing circuit 34, digital scan converter (simply called "DSC(D)" hereinafter) 35, and a mapping circuit 36 for performing color flow mapping processing.

The sending/receiving circuit 31 forms one ultrasonic beam to be sent/received by the ultrasonic transducer array 11 and outputs a received beam signal created from the obtained ultrasonic echo signal to both of the subsequent B-mode processing circuit 32 and the Doppler processing circuit 34. Furthermore, the sending/receiving circuit 31 outputs rotational angle information of an ultrasonic beam for radial scanning to the subsequent DSC (B) 33 and DSC (D) 35 as rotational angle data. The B-mode processing circuit 32 performs publicly known processing, such as logarithmic amplification, envelop detection and A/D conversion, on a received beam signal. Then, the B-mode processing circuit 32 converts the ultrasonic signal to digital echo data and outputs. The DSC(B) 33 converts the echo data in a polar coordinate system to the one in an orthogonal coordinate system for outputting to the monitor 6 and outputs. The Doppler processing circuit 34 performs publicly known processing, such as phase detection, A/D conversion, moving target indicator (MTI) filtering and self-correlation, on the received beam signal so that a moving component, that is, a bloodstream component, in tissue can be extracted by using Doppler effect. Then, the Doppler processing circuit 34 creates and outputs color data for coloring the position of the bloodstream in an ultrasonic tomographic image. The MTI filter removes an unnecessary component based on the slow movement due to various factors such as heartbeats and peristalses. The DSC (D) 35 converts the color data in the polar coordinate system to the orthogonal coordinate system for outputting to the monitor 6 and outputs. The mapping circuit 36 superposes color data output by the DSC(D) 35 on the bloodstream part on the image data of the ultrasonic tomographic image output by the DSC(B) and outputs image data including the color data.

The image processing circuit 27 includes a cube data creating circuit 41, a color part extracting circuit 42, and a three-dimensional constructing circuit 43. The cube data creating circuit 41 reads image data and position and orientation data written in the HDD 6 and creates voxel data, that is, cube data CD having a three-dimensional grid address space. The color part extracting circuit 42 extracts the colored part from the cube data CD. The three-dimensional image constructing circuit 43 constructs a three-dimensional image based on the data of the extracted colored part and outputs three-dimensional image data.

As shown in Fig. 2, the electronic radial scanning type ultrasonic endoscope 2 according to this embodiment mainly includes an endoscope operating portion 13 and an endoscope inserting portion 14. The endoscope inserting portion 14 is inserted to the tube cavity within the body cavity having many curbs, such as the stomach, the gullet and the colon. Therefore, the endoscope inserting portion 14 contains a flexible material. The endoscope operating portion 13 has a bending knob 15 for performing bending operation. A user changes the direction of the distal end of the endoscope inserting portion 14 by bending the endoscope inserting portion 14 by moving a wire, not shown, of the endoscope inserting portion 14 back and forth by rotating the bending knob 15.

Furthermore, the distal end of the endoscope inserting portion 14 has an ultrasonic transducer array 55. The ultrasonic transducer array 55 has a sending coil 51, a lighting window 52 for illuminating inside of the tube cavity, an optical observation window 53 for observation, and many strip-shaped ultrasonic transducers 54 around the distal end of the endoscope inserting portion 14 in a ring-shape. More specifically, the multiple ultrasonic transducers 54 are provided in a ring-shape at a predetermined position of an inserting axis at the distal end of the inserting portion 14 about the inserting axis. A signal line 56 is connected to each of the ultrasonic transducers 54 in the ultrasonic transducer array 55. The signal lines 56 are connected to the ultrasonic processing portion 4. A pulse-shaped send driving voltage for driving the ultrasonic transducers 54 and ultrasonic echo signals from the ultrasonic transducers 54 are sent and received through the signal line 56. Ultrasound is sent and received by the multiple ultrasonic transducers so that the electronic radial scanning can be performed ultrasonically on a plane perpendicular to the inserting axis, which will be described later.

The sending coil 51 includes a complex of multiple solenoid coils in multiple directions therearound. Thus, when the sending coil 51 is excited, anisotropic magnetic fields, that is, multiple magnetic fields having different directions from each other can be caused.

As shown in Fig. 3, the sending/receiving circuit 31 includes a send driving voltage generating circuit 61, a send delay circuit 62, a send switching circuit 63, a receive switching circuit 64, an amplifying circuit 65, a receive delay circuit 66, an adding circuit 67 and a send/receive controller 68.

The send driving voltage generating circuit 61 generates pulse-shaped send driving voltage. The send delay circuit 62 applies and outputs delays different for each signal line to the send driving voltage. The send switching circuit 63 sequentially selects a predetermined multiple number of ultrasonic transducers 54 involved in the generation of an ultrasonic send beam and outputs a send driving voltage. The receive switching circuit 64 sequentially selects ultrasonic echo signals from the multiple ultrasonic transducers 54 involved in the generation of the send beam and outputs the ultrasonic echo signals to the subsequent amplifying circuit 65. The amplifying circuit 65 amplifies the ultrasonic echo signals from the receive switching circuit 64. The receive delay circuit 66 outputs the amplified ultrasonic echo signals by applying the same delay as the delay to the send driving voltage in the send delay circuit 62 thereto. The adding circuit 67 generates and outputs a receive beam signal corresponding to one sound ray by adding the delayed ultrasonic echo signals. The send/receive controller 68 outputs switching control signals for controlling circuits within the ultrasonic signal processing circuit 24 and rotational angle data, which will be described later.

Next, the operations will be described.

[1] An operation in the sending/receiving circuit 31 will be described for outputting signals and data relating to an original image (signals and data required for creating image data of an ultrasonic tomographic image on which color data is superposed) to a synchronous writing circuit.

First, the operation of the sending/receiving circuit 31 will be described.

A send driving voltage generated in the send driving voltage generating circuit 61 is delayed properly by the send delay circuit 62 and is supplied to the multiple ultrasonic transducers 54 selected by the send switching circuit 63. Here, the send delay circuit 62 selects multiple ultrasonic transducers 54 aligned in series by a switching control signal from the send/receive controller 68. Furthermore, the send delay circuit 62 applies a large delay to a send driving voltage of the ultrasonic transducer 54 at center in the alignment. The magnitude of the delay to be applied to the send driving voltage is reduced as the given ultrasonic transducer 54 leaves the center of the alignment. These ultrasonic transducers 54 convert respective send driving voltages to ultrasound with electroacoustic conversion. Each ultrasound forms one send beam by using the delay. The send/receive controller 68 causes the send switching circuit 63 to select the ultrasonic transducers 54 such that ultrasonic beams can rotate in a direction indicated by an arrow RS (in the direction of radial scanning) sequentially through switching control signals. Thus, a so-called radial scanning, which scans a section perpendicular to the inserting axis of the ultrasonic endoscope 2, can be performed.

The ultrasonic transducer array 55 simultaneously sends and receives ultrasound and performs radial scanning. Furthermore, the ultrasonic transducer array 55 converts ultrasonic echo of the scanned plane to electric signals and supplies the electric signals to the receive switching circuit 64 in the sending/receiving circuit 31 as ultrasonic echo signals. The receive switching circuit 64 selects the same multiple number of ultrasonic transducers 54 as the number of those selected by the send switching circuit 63 in response to a switching control signal from the send/receive controller 68. Then, the receive switching circuit 64 outputs the ultrasonic echo signals from the selected ultrasonic transducers 54 to the amplifying circuit 65. The ultrasonic echo signals are amplified in the amplifying circuit 65 and are delayed properly by the receive delaying circuit 66. Then, the ultrasonic echo signals are added in the adding circuit 67, and one ultrasonic receive beam signal can be obtained. The receive beam signal is output to the B-mode processing circuit 32 and the Doppler processing circuit 34.

The send/receive controller 68 outputs a switching control signal to the send switching circuit 63 and the receive switching circuit 64 based on information on which ultrasonic transducer to be switched. On the other hand, the sending/receiving controller 68 outputs as rotational angle data the rotational angle for the radial scanning performed by the ultrasonic transducer array 55 to the DSC(B) 33 and the DSC(D) 35.

Next, the operation of the circuits following the sending/receiving circuit 31 will be described.

The B-mode processing circuit 32 performs publicly known processing such as logarithmic amplification, envelop detection and A/D conversion, on a received beam signal in order to convert an ultrasonic signal to digital echo data.

Th DSC(B) 33 converts the echo data in the polar coordinate system to the one in the orthogonal coordinate system for outputting to the monitor 6 based on the rotational angle data and creates and outputs image data of the ultrasonic tomographic image to the mapping circuit 36.

The Doppler processing circuit 34 performs publicly known processing, such as phase detection, A/D conversion, moving target indicator (MTI) filtering and self-correlation, on the received beam signal so that a moving component, that is, a bloodstream component, in tissue can be extracted by using Doppler effect. Then, color data is created and is output for coloring the position of the bloodstream in the ultrasonic tomographic image. Here, the color data is a hue corresponding to the speed of the bloodstream part.

The DSC(D) 35 converts color data in the polar coordinate system to one in the orthogonal coordinate system for outputting to the monitor 6 based on the rotational angle data and creates and outputs data of the bloodstream component to the mapping circuit 36.

The mapping circuit 36 superposes color data output by the DSC(D) 35 onto the bloodstream part on the image data of the ultrasonic tomographic image output by the DSC(B) 33. Then, the mapping circuit 36 outputs the image data of the ultrasonic tomographic image superposing color data thereon to the synchronous writing circuit 25.

[2] The operation for outputting signals and data relating to positions and orientations (also called "position/orientation data, hereinafter) to the synchronous writing circuit 25 will be described.

The position/orientation detecting portion 3 outputs a coil exciting signal, which is an alternate signal, to the sending coil 12 at the distal end of the ultrasonic endoscope 2. The frequency of the coil exciting signal is different in each winding direction of a lead of each solenoid coil of the sending coil 12. Thus, an alternate magnetic field excited by the frequency different in each direction of each solenoid coil is caused between a part to be examined and the distal end of the endoscope inserting portion 14.

Each of the receiving coil 21 outputs current generated by the magnetic field to the position/orientation detecting portion 3.

The position/orientation detecting portion 3 converts the current to voltage and resolves the frequency. Thus, the difference in winding direction of the lead of the solenoid coil of the sending coil 12 is resolved based on the magnetic field. Then, the position/orientation data of the sending coil 12 expressed in the fixed coordinate system of the receiving coil 21 is calculated and is output to the synchronous writing circuit 25 in the ultrasonic processing portion 4.

The position and orientation data can be calculated as follows.

Position and Orientation Data:

Position of Sending Coil 12: (Dx, Dy, Dz)

Orientation of Sending Coil 12: ($\psi$, $\theta$, $\phi$) [Euler Angles]

[3] The operation will be described for displaying a three-dimensional image based on data output to the synchronous writing circuit 25.

The synchronous writing circuit 25 writes associated image data and position/orientation data synchronously to HDD 26.

The cube data creating circuit 41 reads out image data and position/orientation data written in the HDD 26, creates cube data CD having a three-dimensional grid address and outputs the cube data CD to the color part extracting circuit 42. The cube data CD and the method for creating cube data CD will be described in section [4]-2.

The color part extracting circuit 42 extracts the colored part from the cube data CD and replaces image data of the other part within the cube data by zero (0), that is, undisplayed data. Then, the processed cube data CD is output to the three-dimensional image constructing circuit 43.

The three-dimensional image constructing circuit 43 constructs a three-dimensional image from the extracted part and outputs the image data to the display circuit 28.

The display circuit 28 converts image data of the three-dimensional image to analog video signals, which can be displayed on the monitor 6 and outputs the analog video signals to the monitor 6. The monitor 6 displays the three-dimensional image.

[4] Practical uses by users will be described.

[4]-1 The recording of signals and data relating to an original image and data relating to positions and or directions into the HDD 26 will be described with reference to Fig. 4.

The processing shown in Fig. 4 is started when a user presses a scanning control key 22 on the keyboard 5 and is controlled by the control circuit 29.

When the control circuit 29 detects that the scanning control key 22 has been pressed, the control circuit 29 implements radial scanning processing. The ultrasonic transducer array 11 performs radial scanning by ultrasonic beams in response to the instruction from the control circuit 29 (S11, where S stands for step).

When the radial scanning is implemented, the receiving coil 21 receives a magnetic-field signal from the sending coil 12, and the position/orientation detecting portion 3 detects the position and orientation of the scanning plane and creates position/orientation data (S12). At the same time, the ultrasonic signal processing circuit 24 creates image data of the ultrasonic tomographic image superposing color data thereon (S13).

Then, the synchronous writing circuit 25 records the associated position/orientation data and image data of the ultrasonic tomographic image superposing color data thereon to the HDD 26 synchronously (S14).

Next, whether the scanning control key 22 has been pressed by the user again or not is determined (S15). If the user has pressed the scanning control key 22 again, the processing goes to the next step S16 (YES). Otherwise, the processing jumps to a step S11, and the above-described steps are repeated.

At a step S16, the control circuit 29 outputs a command for terminating the radial scanning, and the ultrasonic transducer array 11 stops the radial scanning by ultrasonic beams in response to the instruction from the control circuit 29.

Here, when the user moves the radial scanning type ultrasonic endoscope 2 back and forth by hand and/or changes the scanned plane by using the bending knob 15, the above-described series of steps is repeated. Thus, the image data of the ultrasonic tomographic image is associated with the position/orientation data, and only the part required for constructing a three-dimensional image is recorded in the HDD 26 in series.

[4]-2 The three-dimensional image creation from the data written in the HDD 26 will be described with reference to Fig. 5.

The processing shown in Fig. 5 is started when a user presses the 3D key 23 on the keyboard and is controlled by the control circuit 29.

When the control circuit 29 detects that the 3D key 23 has been pressed, the cube data creating circuit 41 reads out image data and position/orientation data of an ultrasonic tomographic image stored in the HDD26 as shown in Fig. 6 in response to the instruction from the control circuit 29 (S21). The cube data creating circuit 41 embeds image data of the ultrasonic tomographic image superposing every piece of color data thereon in a memory space having a three-dimensional grid address. The embedding method will be described later. In this way, cube data CD as shown in Fig. 6 is created (S22). Here, since the ultrasonic tomographic images are not always parallel to each other, the cube data creating circuit 41 gets the average of the overlapped part and interpolates the loose part. Thus, the data density can be regular.

Next, the color part extracting circuit 42 extracts the colored part (bloodstream part) from the cube data CD and replaces image data of the other part in the cube data by zero (0), that is, by undisplayed data (S23).

The three-dimensional image constructing circuit 43 performs publicly known three-dimensional image processing such as coordinate conversion, hidden surface removal and shading and the like and constructs a three-dimensional image expressing the bloodstream traveling as shown in Fig. 7 from the extracted part (S24).

The display circuit 28 converts the image data of the three-dimensional image to analog video signals, which can be displayed on the monitor 6, and outputs (S25). As a result, the monitor 6 displays the three-dimensional image expressing the bloodstream traveling as shown in Fig. 7. A great vessel 72 and a small vessel 73 are displayed on a screen 71 of the monitor 6.

[4]-3 The method for creating cube data will be described in detail (which is supplemental description on the step S21 in Fig. 5).

A user moves the distal end of the endoscope by hand and obtains ultrasonic tomographic images in series. Thus, cube data CD having three-dimensional addresses can be created. However, in order to do so, the positions of the ultrasonic tomographic images captured in series in the space must be expressed as coordinates in the coordinate system fixed into the space by using the data available to the cube data creating circuit 41. The method will be described below.

The receiving coil 21 is fixed in the space, and the sending coil 12 is fixed near the distal end of the endoscope 2. Here, as shown in Fig. 8, an orthogonal coordinate system O-xyz fixed to the receiving coil 21 and an orthogonal coordinate system O'-x'y'z' fixed to the sending coil 12 are introduced. The addresses of the cube data CD are expressed by the coordinates in the orthogonal coordinate system O-xyz.

The origin, the coordinate axes and the unit vector are plotted as shown in Table 1.

Table 1

| Names of Orthogonal Coordinate Systems | Plotting of Origin and Coordinate Axes | Unit Vectors | Remarks |
|---|---|---|---|
| O-xyz | O: An arbitrary point in a space fixed to the receiving coil Coordinate Axes: Arbitrary directions fixed to the receiving coil | *i, j, k* | Fixed to the receiving coil (fixed in the space) |
| O'-x'y'z' | O': A fixed point in the sending coil (which substantially coincides with the center of ultrasonic tomographic images since the sending coil is provided near the transducers) Coordinate Axes: The axes x' and y' are plotted so as to coincide with the horizontal and vertical directions, respectively, of ultrasonic tomographic images. | *i', j', k'* | Fixed to the sending coil (which moves when the user moves the distal end of the endoscope). |

Here, A is an arbitrary point on the ultrasonic tomographic image 78 (for example, a part of the bloodstream 79) in Fig. 8. Now, the method will be described in which the cube data creating circuit 41 uses known data obtained from the HDD 26 to express the coordinates of the point A in the orthogonal coordinate system O-xyz of the receiving coil 21 fixed to the space.

The known data includes a position data (horizontally "a", vertically "b") of the point A within an image, position data (Dx, Dy, Dz), and orientation data ($\psi$, 0, $\phi$) [Euler angles] on the scanned plane by position 0' of the sending coil 12 output by the position/orientation detecting portion 3 in the orthogonal coordinate system O-xyz.

Therefore, for the above-mentioned expression, the position of the point A may be expressed as the primary coupling of unit vectors (i, j, k) of the orthogonal coordinate system O-xyz by using these kinds of known data.

Here, the relationships below are obtained:

$$r = R + r' \qquad\qquad \text{Expression 1}$$

$$r' = ai' + bj' \qquad\qquad \text{Expression 2}$$

$$R = Dxi + Dyj + Dzk \qquad\qquad \text{Expression 3}$$

where *r* is the positional vector of the point A in the O-xyz coordinate system, *r'* is the positional vector of the point A in the O'-x'y'z' coordinate system, and *R* is the positional vector of the point O' in the O-xyz coordinate system.

By substituting Expressions 2 and 3 for Expression 1, the following expression can be obtained:

$$r = Dxi + Dyj + Dzk + ai' + bj' \qquad\qquad \text{Expression 4}$$

According to Expression 4, (i', j', k') (where i', j' and k' are vectors) may be expressed by (i, j, k) (where i, j, k are vectors) by using known amounts. When the rotational matrix Tx(ψ), Ty(θ) and Tz(φ) defined from the Euler angles (ψ, θ, φ) are used, the following expression 5 can be obtained.

$$[i', j', k'] = [i, j, k] Tx(\psi) Ty(\theta) Tz(\phi) \qquad \text{Expression 5}$$

where **Tx**(ψ), **Ty**(θ) and **Tz**(φ) are the rotational matrix defined from the Euler angles (ψ, θ, φ).
Here, the rotational matrix is defined by the following expressions:

**Expression 6**

$$Tx(\psi) = \begin{pmatrix} 1 & 0 & 0 \\ 0 & \cos\psi & -\sin\psi \\ 0 & \sin\psi & \cos\psi \end{pmatrix}$$

**Expression 7**

$$Ty(\theta) = \begin{pmatrix} \cos\theta & 0 & \sin\theta \\ 0 & 1 & 0 \\ -\sin\theta & 0 & \cos\theta \end{pmatrix}$$

**Expression 8**

$$Tz(\phi) = \begin{pmatrix} \cos\phi & -\sin\phi & 0 \\ \sin\phi & \cos\psi & 0 \\ 0 & 0 & 1 \end{pmatrix}$$

When i' (where i' is a vector) and j' (where j' is a vector) obtained by substituting Expressions 6 to 8 for Expression 5 are substituted for Expression 4, the position r (where r is a vector) of the point A may be expressed by the primary coupling of unit vectors (i, j, k) (where i, j, and k are vectors) in the O-xyz coordinate system by using the known data a, b, Dx, Dy, Dz, ψ, θ and φ. That is, the arbitrary point A on the ultrasonic tomographic image can be expressed in the coordinate system fixed in a space.

Thus, the cube data creating circuit 41 substitutes the coordinates (a, b) on the ultrasonic tomographic image and the position/orientation data (Dx, Dy, Dz) and (ψ, θ, φ) for Expressions 4 and 5. Then, image data at the points are filled in the space in cube data, and the averaging of the overlap part and the interpolating of the loose part can be performed. As a result, cube data can be created.

As described above, according to this embodiment, an ultrasonic three-dimensional image can be constructed easily from voxel data.

Furthermore, according to this embodiment, for displaying the tissue including a tumor by an ultrasonic three-dimensional image in the in-body-cavity ultrasonic diagnosis field, an ultrasonic diagnosis apparatus, which can obtain good image data having no distortion by the easy operation, can be realized.

Furthermore, according to this embodiment, in the in-body-cavity ultrasonic field, for displaying the bloodstream and the tissue including the bloodstream, an ultrasonic diagnosis apparatus, which can obtain the bloodstream information using Doppler effect by the easy operation, can be realized.

According to this embodiment, in a case of an esophageal varix, for example, the running of the bloodstream, which is difficult to represent in a conventional ultrasonic endoscope, can be displayed by coloring by using color data obtained by slowly retracting an endoscope inserting portion inserted in the gullet by a user. This is effective for diagnosis of an esophageal varix requiring the diagnosis of the state of the varix in the bloodstream and/or the traveling of the bloodstream bypass, for example.

The associated image data and position/orientation data are recorded in the HDD 26. Therefore, a three-dimensional image can be constructed for reviews after an examination.

Next, variation examples will be described.

A three-dimensional image may be constructed by combining the section 74 and the in-body-cavity organ surface 75 only by using black-and-white ultrasonic tomographic images, as shown in Fig. 9, instead of color data. Alternatively, the other kinds of three-dimensional image can be constructed. In Fig. 9, the reference numeral 76 indicates a tumor. Therefore, a three-dimensional image without distortion due to the twist of the flexible shaft can be observed more easily than the cases using the conventional mechanical radial scanning type ultrasonic endoscope and the Kolvex scanning type ultrasonic endoscope.

The synchronous writing circuit 25 records in the HDD 26 image data of an ultrasonic tomographic image superposing color data thereon, and the color part extracting circuit 42 extracts only the color data. However, in order to display a three-dimensional image of only the traveling of the bloodstream like in this embodiment, the color data can be only recorded in the HDD 26 from the beginning.

Furthermore, the positions of the sending coil 12 and the receiving coil 21 may be reversed. Then, the sending coil 12 may be spatially fixed while the receiving coil 21 may be provided at the distal end of the endoscope inserting portion of the radial scanning type ultrasonic endoscope 2. In this case, the address of the cube data may be expressed in the orthogonal coordinate system O'-x'y'z'.

In addition, in this embodiment, data relating to the orientation output by the position/orientation detecting portion 3 expresses three angles of Euler angles $\psi$, $\theta$, $\phi$. However, the other kinds of data may be used such as data on the direction of the axis having a lead of the solenoid coil therearound of the sending coil 12.

[Second Embodiment]

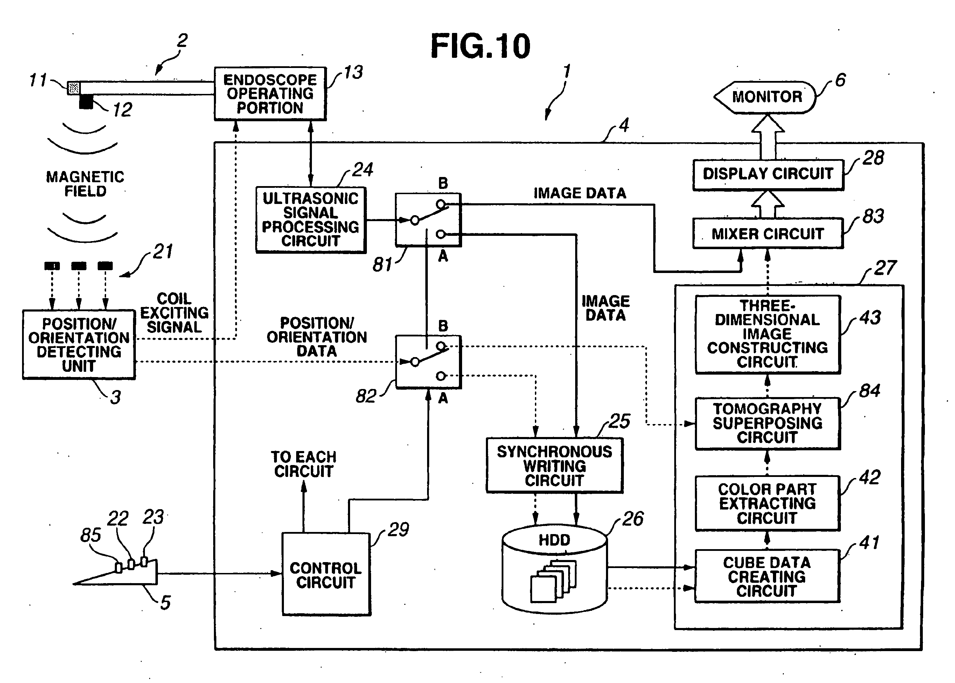

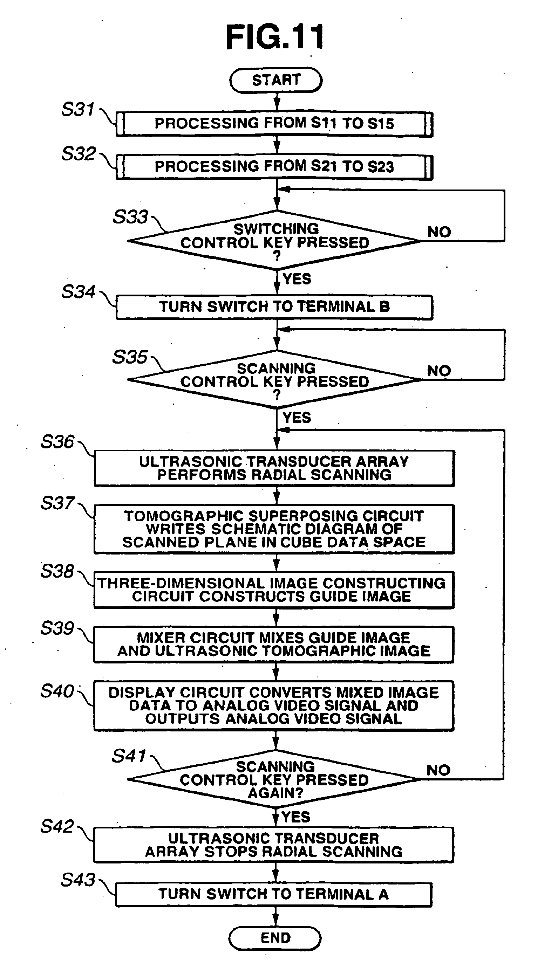

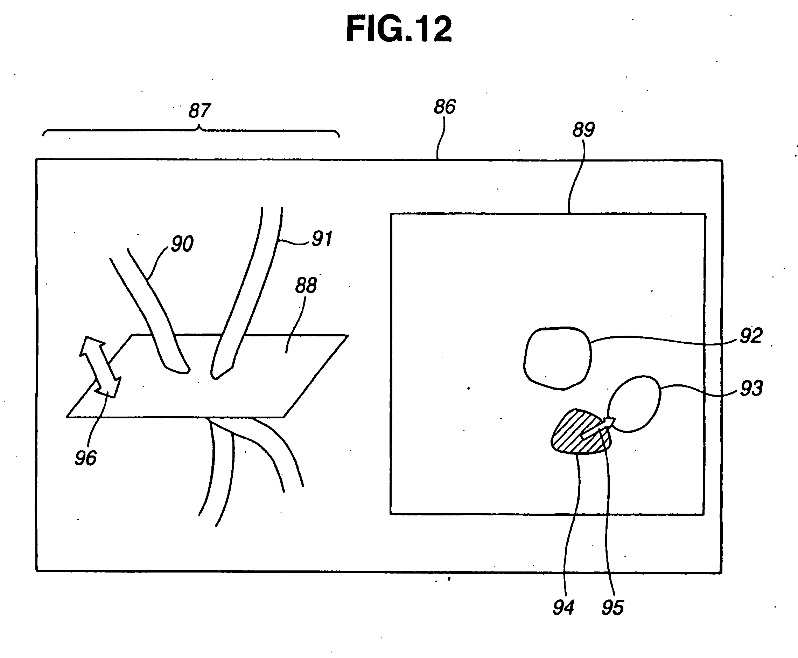

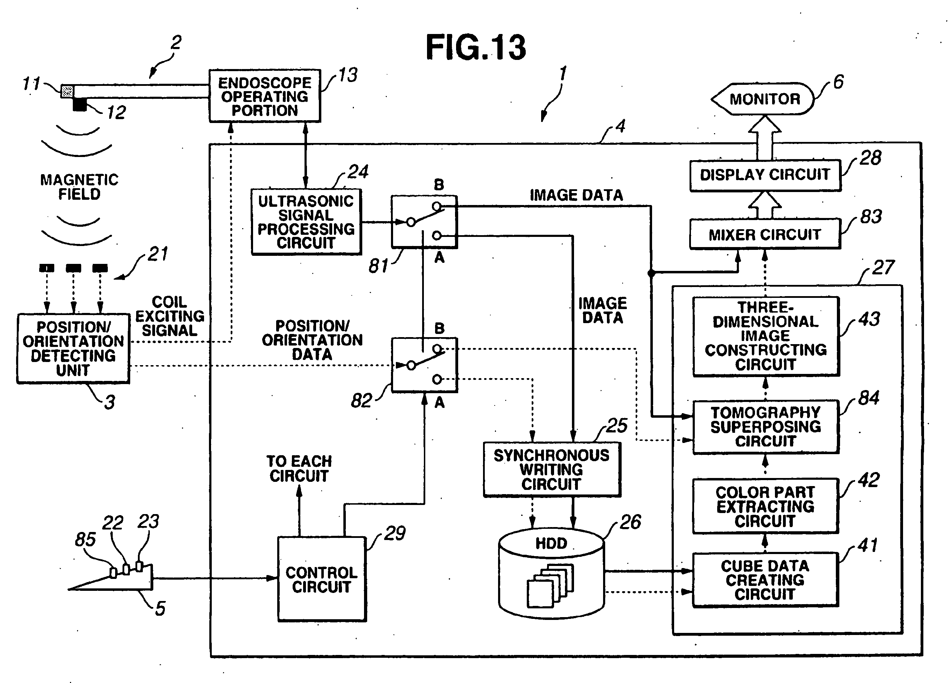

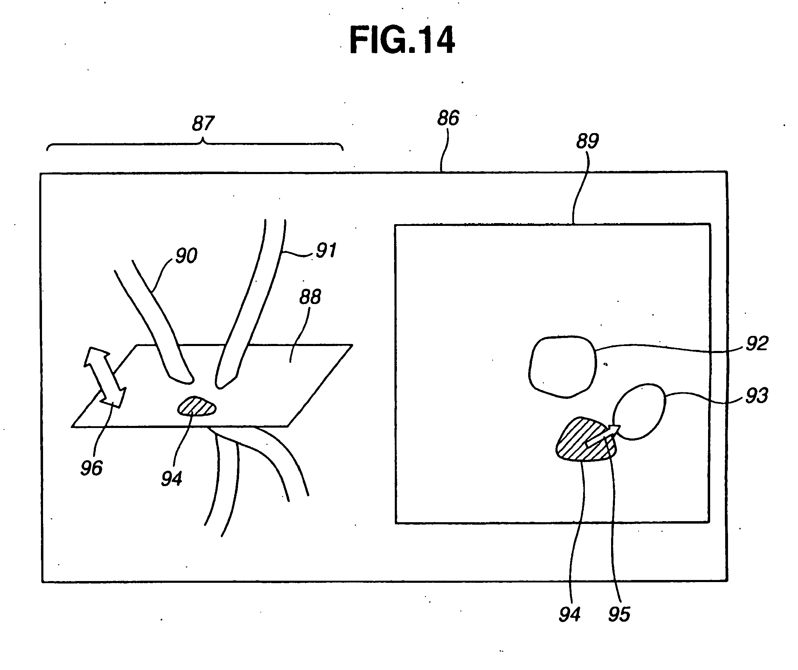

Figs. 10 to 14 show a second embodiment of the invention. Fig. 10 is a diagram for explaining the entire construction of an ultrasonic diagnosis apparatus according to the second embodiment of the invention. Fig. 11 is a flowchart showing an operation flow of the ultrasonic diagnosis apparatus according to the second embodiment. Fig. 12 is a diagram showing a monitor screen example according to the second embodiment. Fig. 13 is a diagram for explaining a variation example of the entire construction according to the second embodiment. Fig. 14 is a diagram showing a variation example of the monitor screen according to the second embodiment.

As shown in Fig. 10, an ultrasonic diagnosis apparatus 1 according to this embodiment has an ultrasonic processing unit 4. The ultrasonic processing unit 4 includes two switches 81 and 82 switched synchronously (where, generally, the terminal A side is ON) and a mixer circuit 83. An image processing circuit 27 has a tomographic image superposing circuit 84 between a color part extracting circuit 42 and a three-dimensional image constructing circuit 43. Furthermore, a switching control key 85 is provided on the keyboard. Every time a user presses the switching control key 85, the switches 81 and 82 are switched from one side to the other.

The other construction is the same as the one of the first embodiment. The same reference numerals are given to the same components as those of the first embodiment, and the description will be omitted here.

In Fig. 10, the arrow having a thick dotted line indicates the flow of signals or data relating to a three-dimensional image. The arrow having a thick solid line indicates the flow of signals or data relating to an original image. The arrow having a thin dotted line indicates the flow of signals or data relating to a position and/or an orientation. The arrow having a thin solid line indicates the flow of the other signals, such as control signals, or data. The open thick arrow indicates signals or data of an image in Fig. 12.

Next, the operation of this embodiment will be described.

When the switches 81 and 82 are turned to the A-side, the position/orientation data are not input to the tomographic image superposing circuit 84. Therefore, the tomographic image superposing circuit 84 outputs the input cube data CD to the three-dimensional image constructing circuit 43 as it is. Therefore, the same operation as the one of the first embodiment is performed.

The operation different from the one of the first embodiment will be described below with reference to Fig. 11

First of all, a user presses the scanning control key 22, and the ultrasonic processing portion 4 then records data in the HDD 26 (S31) like the steps S11 to S15 of the first embodiment. Here, the switches 81 and 82 are turned to the terminal A side.

Next, the user presses the 3D key 23 like the first embodiment, and the cube data creating circuit 41 and the color part extracting circuit 42 perform the same steps (S32) as the step S21 to S23 of the first embodiment.

Then, whether the switching control key 85 has been pressed or not is determined (S33). If NO at the Step S33, that is, if the switching control key 85 has not been pressed, no processing is performed. If YES at the step S33, that is, if the switching control key 85 has been pressed, the processing goes to a step S34. At the step S34, the switches 81 and 82 are turned from the terminal A side to the terminal B side.

Then, whether the scanning control key 22 has been pressed by the user or not is determined (S35). If NO at the step S35, that is, if the scanning control key 22 has not been pressed, no processing is performed. If YES at the step S35, that is, if the scanning control key 22 has been pressed, the processing goes to a step S36.

At the step S36, the ultrasonic transducer array 11 performs radial scanning by an ultrasonic beam again in response to an instruction from the control circuit 29, and the processing goes to a step S37.

At the step S37, the tomographic image superposing circuit 84 writes the scanned plane by the electronic radial scanning type ultrasonic endoscope 2 in the space of cube data CD in real time as a plate-like schematic diagram, based on serially input position/orientation data. The method for writing the scanned plane will be described later.

Then, at a step S38, the three dimensional image constructing circuit 43, which is combining means, constructs a three-dimensional image (also called "guide image" hereinafter) having a schematic diagram of a scanned plane on the traveling of the bloodstream based on the bloodstream part and plate-like schematic diagram data in the cube data. In other words, the three-dimensional image constructing circuit 43, that is combining means, creates a guide image by combining an ultrasonic three-dimensional image of the bloodstream and the schematic diagram showing the position or the orientation of the ultrasonic tomographic image. This step is performed in real time.

At a step S39, the mixer circuit 83 mixes a guide image on the left side and an ultrasonic tomographic image on the right to create a screen as shown in Fig. 12.

At a step S40, the display circuit 28 converts image data on a screen mixed by the mixer circuit 83 to analog video signals to be displayed on the monitor 6 and outputs the analog video signals to the monitor 6. At a step S41, the monitor 6 displays the guide image and the ultrasonic tomographic image on the left and on the right, respectively, as shown in Fig. 12.

At the step S41, whether or not the user has pressed the scanning control key 22 again or not is determined. If YES at the step S41, that is, if the scanning control key 22 has been pressed, the radial scanning using an ultrasonic beam is terminated in response to an instruction of the control circuit 29, and the processing goes to a next step S42. Otherwise, that is, if NO at the step S41, the processing goes to the step S36, where the subsequent steps are performed again.

At a step S42, the ultrasonic transducer array 11 stops the radial scanning using an ultrasonic beam in response to an instruction from the control circuit 29. At a step S43, the switches 81 and 82 are turned from the terminal B to the terminal A.

Here, the series of steps from S31 to S43 is performed while the user is inserting the endoscope inserting portion 14 into a part to be examined. A guide image 87 on the left side of the monitor screen 86 in Fig. 12 shows a schematic diagram 88 showing an ultrasonically scanned plane (indicating the position and orientation of a tomographic image). An ultrasonic tomographic image 89 corresponding to the schematic diagram obtained by the scanned plane appears on the right side of the monitor screen 86 in Fig. 12. In Fig. 12, the reference numeral 90 indicates blood A, and the reference numeral 91 indicates blood B. The reference numeral 92 indicates colored blood A. The reference numeral 93 indicates colored blood B. The reference numeral 94 indicates a tumor, and the reference numeral 95 indicates an infiltration direction. Furthermore, the schematic diagram 88 itself on the left hand side moves within a three-dimensional image in a direction indicated by an arrow 96 in accordance with (in synchronization with) the movement of the distal end of the ultrasonic endoscope 2 based on position/orientation data serially output by the position/orientation detecting portion 3. In other words, in accordance with the change in data sequentially output from the position/orientation detecting portion 3, the combination state of the schematic diagram and the three-dimensional image changes sequentially. The ultrasonic tomographic image on the right side also changes correspondingly. This operation is performed in real time.

The detail of the method for writing a scanned plane into cube data CD at the step S37 will be described below.

An ultrasonic tomographic image is rectangular, and the schematic diagram of the scanned plane can be represented by a parallelogram having sides in a unit vector i' direction (where i' is a vector) and j' direction (where j' is a vector) in the orthogonal coordinate system O'-x'y'z' in the cube data. Therefore, the tomographic image superposing circuit 84 substitutes the sequentially input position/orientation data for Expression 5. Here, i' and j' (where i' and j' are vectors) can be expressed as the primary coupling of the unit vectors i, j and k (where i, j and k are vectors) in the orthogonal coordinate system O-xyz. Therefore, by using this, the schematic diagram of the parallelogram can be written in the space of the cube data easily.

The other operation is the same as the one according to the first embodiment.

As described above, according to the ultrasonic diagnosis apparatus of this embodiment, a schematic diagram showing the position or orientation includes superposed ultrasonic tomographic image data. Therefore, the positional relationship between the lesion such as a tumor and the bloodstream, for example, can be identified more easily.

In this way, according to this embodiment, the degree of infiltration to a blood vessel can be diagnosed more easily by moving the endoscope inserting portion and by, at the same time, selecting the position where the lesion such as a tumor, for example, and the bloodstream are the closest to each other on the ultrasonic tomographic image on the right side and by observing the ultrasonic tomographic image.

The scanned plane is shown on the guide image in real time. Therefore, the scanned plane by the ultrasonic transducer array for the bloodstream can be identified more easily. Thus, the lesion can be rendered more easily. The degree of reach in depth toward the bloodstream of the lesion such as a tumor and/or the degree of advance along the bloodstream can be less missed. This is useful for prognoses regarding a spread, for example.

Since the guide image displaying the bloodstream three-dimensionally can be easily compared with the ultrasonic tomographic image, a cure effect, such as where the reduction of the lesion in size in the change occurs, can be recognized more easily based on the histological difference in the lesion than the comparison based on volume measuring, for example. The other advantages are the same as those of the first embodiment.

Next, the variation examples will be described.

The schematic diagram is a simple parallelogram in the description above. However, as shown in Fig. 13, the image data output from the switches 81 and 82 may be output in parallel to the mixer circuit 83 and the tomographic image superposing circuit 84. Thus, the schematic diagram may be a parallelogram superposing image data of ultrasonic tomographic images as shown in Fig. 14, instead of a simple parallelogram. With this construction, the positional relationship of a lesion such as a tumor and the bloodstream can be clearer. In Fig. 13, the arrow having a thick dotted line indicates the flow of signals or data relating to a three-dimensional image. The arrow having a thick solid line indicates the flow of signals or data relating to an original image. The arrow having a thin dotted line indicates the flow of signals or data relating to a position and/or an orientation. The arrow having a thin solid line indicates the flow of the other signals, such as control signals, or data. The open thick arrow indicates signals or data of an image in Fig. 14.

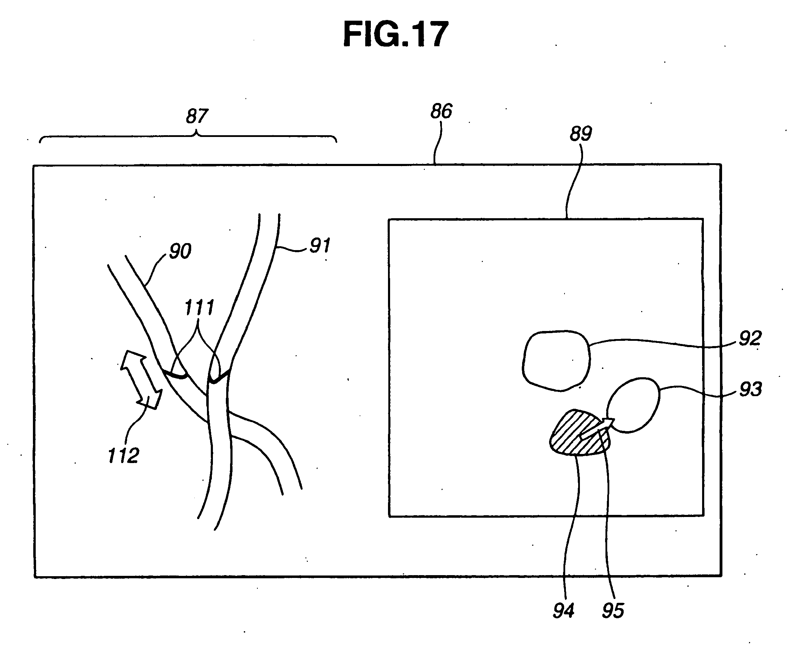

While the schematic diagram is a parallelogram in the description, the schematic diagram may be a line, which will be described later with reference to Fig. 17.

[Third Embodiment]

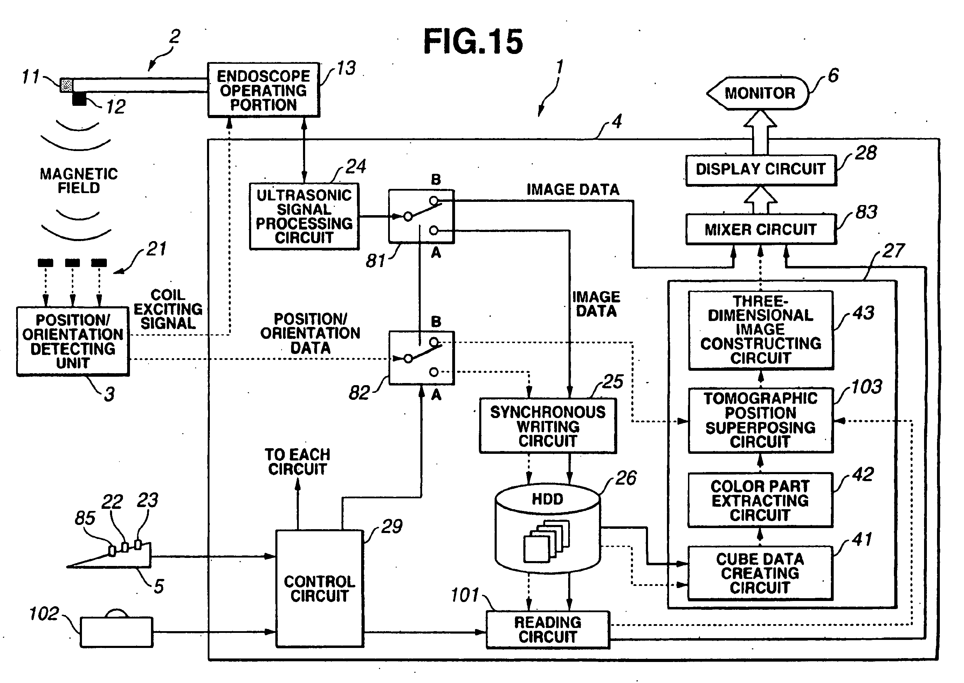

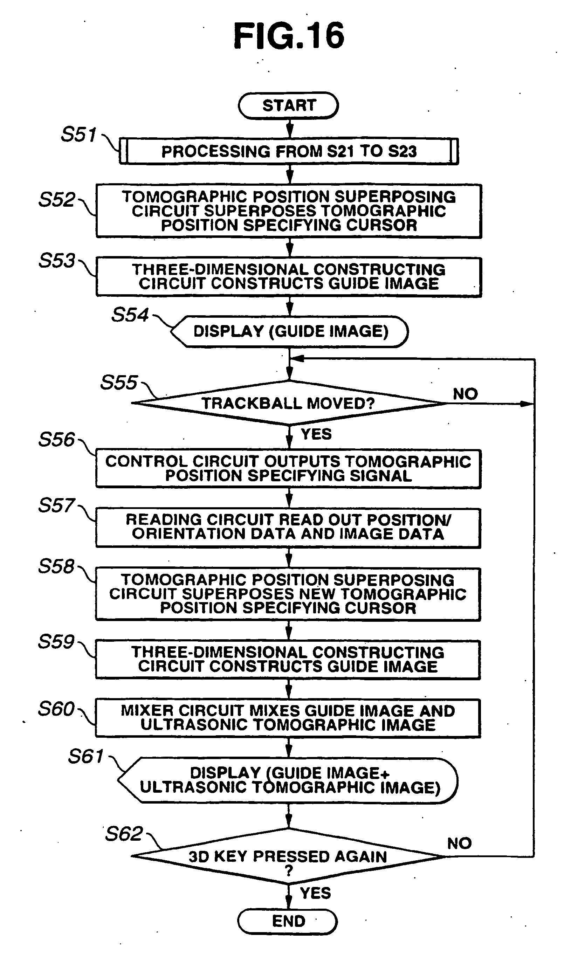

Figs. 15 to 17 show a third embodiment of the invention. Fig. 15 is a diagram for explaining the entire construction of an ultrasonic diagnosis apparatus according to a third embodiment of the invention. Fig. 16 is a flowchart showing an operation flow of the ultrasonic diagnosis apparatus according to the third embodiment. Fig. 17 is a diagram showing a monitor screen display example according to the third embodiment.

As shown in Fig. 15, an ultrasonic diagnosis apparatus 1 according to this embodiment has a reading circuit 101 in an ultrasonic processing unit 4. A trackball 102 is externally provided. A control circuit 29 outputs tomographic position specifying signals to the reading circuit 101 based on the output of the trackball 102. The reading circuit 101 searches and reads image data and position/orientation data from the HDD 26 based on the tomographic position specifying signal and outputs image data and position/orientation data to a mixer circuit 83 and a tomographic position superposing circuit 103, respectively.

The other construction is the same as those of the first and second embodiments. The same reference numerals are given to the same components as those of the first and second embodiments, and the description will be omitted.

In Fig. 15, the arrow having a thick dotted line indicates the flow of signals or data relating to a three-dimensional image. The arrow having a thick solid line indicates the flow of signals or data relating to an original image. The arrow having a thin dotted line indicates the flow of signals or data relating to a position and/or an orientation. The arrow having a thin solid line indicates the flow of the other signals, such as control signals, or data. The open thick arrow indicates signals or data of an image in Fig. 17.

Next, the operation of this embodiment will be described.

The operation of this embodiment mainly includes reviews during and after examination of a part to be examined by an operator.

[1] Operation During Examination
The operation during examination is the same as that of the second embodiment, and the description will be omitted here.

[2] Operation After Examination

The operation after examination will be described with reference to Fig. 16.

The processing in Fig. 16 is started when a user presses the 3D key 23. First of all, at a step S51, the cube data creating circuit 41 and the color part extracting circuit 42 perform the same steps (S51) as the steps S21 to S23 according to the first embodiment. Next, at a step S52, the tomographic position superposing circuit 103 superposes a line (tomographic position specifying cursor) 111 indicating a scanned plane in which an ultrasonic tomographic image is obtained on an extracted bloodstream in cube data, that is, on the part where the bloodstream runs, as shown in the guide image 87 on the left side of Fig. 17. In the beginning of the processing, the line 111 may be superposed on any position of the bloodstream part.

Next, at a step S53, the three-dimensional image constructing circuit 43 constructs the guide image 87 superposing the tomographic position specifying cursor 111 thereon. Then, the guide image 87 superposing the tomographic position specifying cursor 111 thereon is displayed on the monitor 6 through the mixer circuit 83 and the display circuit 28 (S54). In the beginning of the processing, the ultrasonic tomographic image 89 has not been displayed on the monitor screen 86 yet.

The user moves the trackball 102 while watching the tomographic position specifying cursor 111 on the guide image 87. At a step S55, whether the trackball 102 has been moved or not is detected.

If NO at the step S55, that is, if the trackball 102 has not been moved, no processing is performed. If YES at the step S55, that is, if the trackball 102 has been moved, the processing goes to a step S56. The control circuit 29 outputs, as a position specifying signal, information on the direction and distance of the movement of the tomographic position specifying cursor 111 on the monitor screen 86 based on the output of the trackball 102 to the reading circuit 101 (S56).

The reading circuit 101 searches the position/orientation data at the position of or near the tomographic position specifying cursor 111 specified by the tomographic position specifying signal from the HDD 26 and reads the position/orientation data and image data of the ultrasonic tomographic image associated therewith (S57). The tomographic position superposing circuit 103 superposes a old tomographic position specifying cursor on the extracted bloodstream part in the cube data, instead of the old tomographic position specifying cursor, based on the position/orientation data (S58). The three-dimensional image constructing circuit 43 constructs the guide image 87 superposing the tomographic position specifying cursor 111 thereon (S59).

Then, the mixer circuit 83 mixes image data of the guide image 87 and the image data of the ultrasonic tomographic image 89 associated with the position/orientation data corresponding to the tomographic position specifying cursor 111 (S60). The guide image superposing the new tomographic position specifying cursor thereon instead of the guide image superposing the old tomographic position specifying cursor thereon and the ultrasonic tomographic image corresponding thereto are displayed on the monitor 6 through the mixer circuit 83 and the display circuit 28 (S61). At a step S62, whether the user has pressed the 3D key again or not is determined. If YES, the processing is ended in response to the instruction of the control circuit 29. Otherwise, the processing jumps to the step S55, and the subsequent steps are performed again.

Therefore, a user uses the trackball 102 and moves the cursor 111 in a direction indicated by the arrow 112, for example so as to move the cursor position to the position to be observed on the guide image 87 on the left side of the monitor screen. Thus, the ultrasonic tomographic image 89 on the right side is changed and is updated in accordance with the movement of the cursor. This state is shown in Fig. 17. The other operation is the same as that of the second embodiment.

As described above, according to this embodiment, a user uses a trackball and moves the cursor position to the position to be observed on the guide image on the left side of the monitor screen so that the ultrasonic tomographic image on the right side is changed and is updated in accordance with the movement of the cursor. Therefore, the range of the lesion such as a tumor can be shown more clearly, and the degree of advance can be diagnosed more easily. Since the processing is performed based on the image data and position/orientation data recorded in the HDD, the diagnosis can be performed more easily during the case review after the examination. The other advantages are the same as those of the second embodiment.

Next, variation examples will be described.

In the above-described embodiment, the reading circuit 101 searches the position/orientation data at the position of or near the tomographic position specifying cursor 111 specified by the tomographic position specifying signal from the HDD 26 and reads the position/orientation data and image data of the ultrasonic tomographic image 89 associated therewith. However, by replacing the reading circuit 101 by an arbitrary tomographic image creating circuit, an ultrasonic tomographic image having a section perpendicular to the bloodstream direction may be newly created from image data recorded in the HDD 26. Generally, the diagnosis for the infiltration to the blood vessel determines the distance from the lesion to the blood vessel. Thus, the diagnosis is preferably performed by using an ultrasonic tomographic image at the section perpendicular to the bloodstream. Therefore, with this construction, the good observation can be implemented by using an ultrasonic tomographic image at the section perpendicular to the running direction of the blood vessel instead of an ultrasonic tomographic image at the section diagonal to the running direction of the blood vessel. Also, in this case, a parallelogram schematic diagram as shown in Fig. 12 may be used instead of the tomographic position specifying cursor 111. Then, when the schematic diagram is moved by the trackball 102, the positional relationship between the ultrasonic tomographic image and the bloodstream can be shown more clearly.

[Fourth Embodiment]

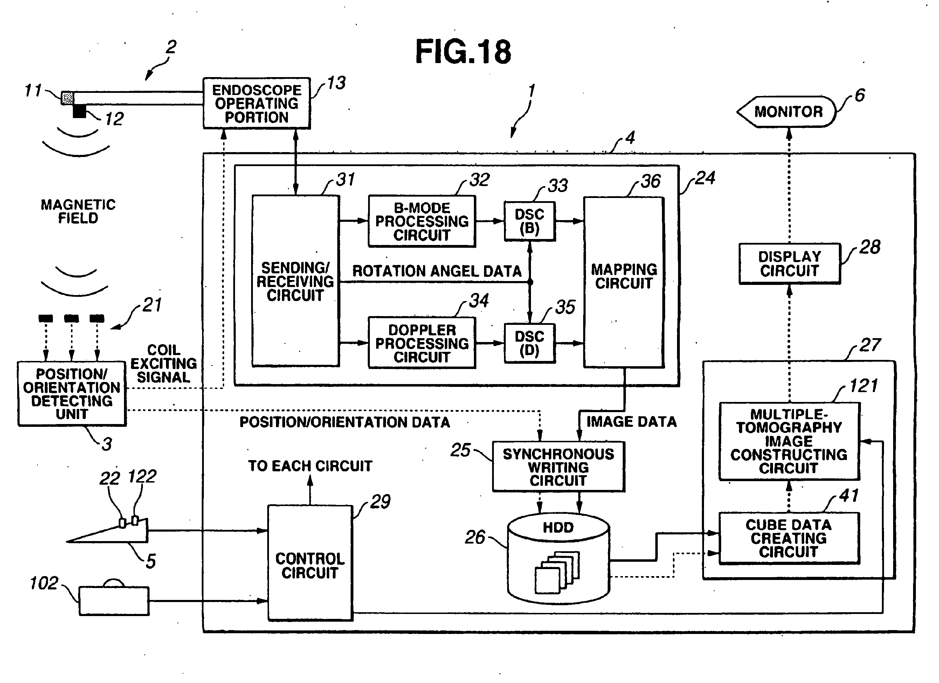

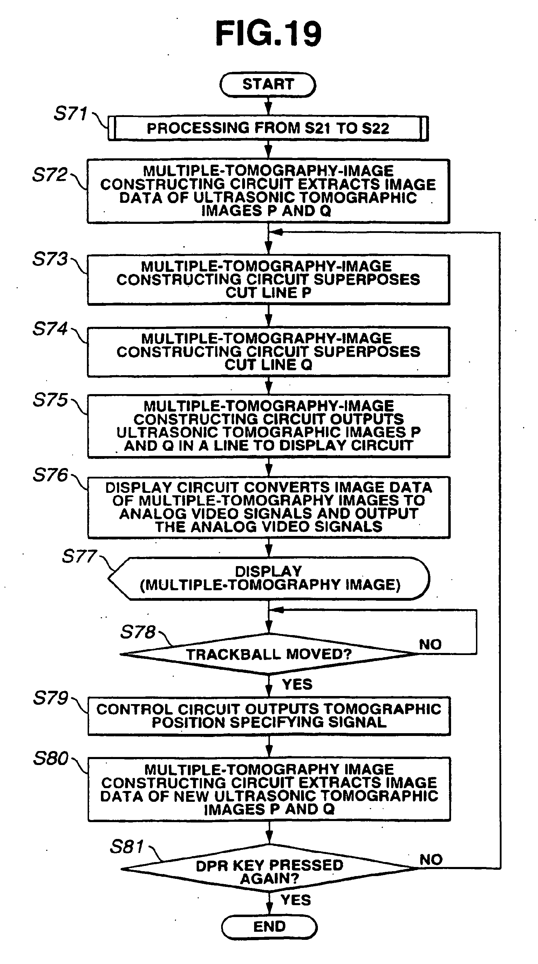

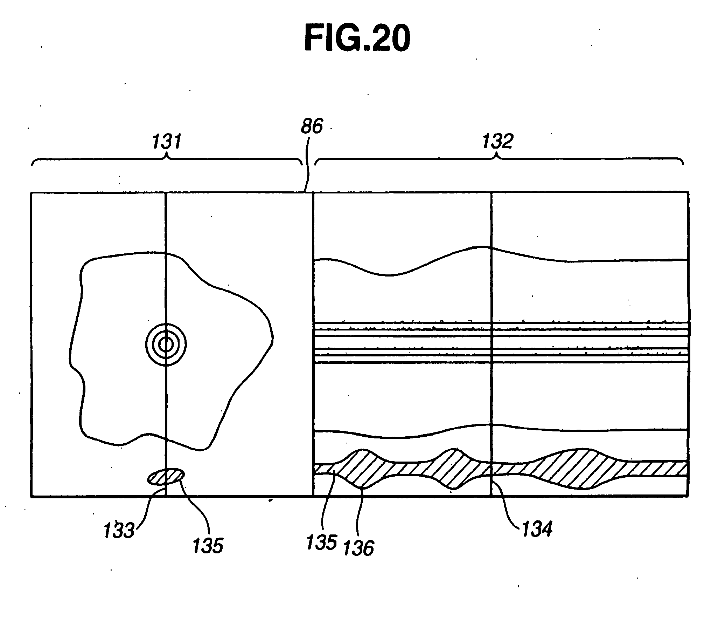

Figs. 18 to 20 show a fourth embodiment of the present invention. Fig. 18 is a diagram for explaining the entire construction of an ultrasonic diagnosis apparatus according to the fourth embodiment of the invention. Fig. 19 is a flowchart showing an operation flow of an ultrasonic diagnosis apparatus according to the fourth embodiment. Fig. 20 is a diagram showing a monitor screen display example according to the fourth embodiment.

As shown in Fig. 18, an ultrasonic diagnosis apparatus 1 according to this embodiment externally has a trackball 102. Instead of the color part extracting circuit 42 and the three-dimensional image constructing circuit 43, a multiple-tomographic-image constructing circuit 121 is provided for constructing multiple ultrasonic tomographic images having different directions from each other. Furthermore, instead of the 3D key 23 on the keyboard 5, a DPR key 122 is provided. A control circuit 29 generates a tomographic position specifying signal based on the output of the trackball 102 and outputs the tomographic position specifying signal to the multiple-tomographic-image constructing circuit 121.

The other construction is the same as the one according to the first embodiment. The same reference numerals are given to the same components as those of the first embodiment, and the description will be omitted here.

In Fig. 18, the arrow having a thick dotted line indicates the flow of signals or data relating to a three-dimensional image. The arrow having a thick solid line indicates the flow of signals or data relating to an original image. The arrow having a thin dotted line indicates the flow of signals or data relating to a position and/or an orientation. The arrow having a thin solid line indicates the flow of the other signals, such as control signals, or data.

Next, the operation of this embodiment will be described.

The operation of this embodiment is the same as that of the first embodiment except for the operations of the trackball 102, the control circuit 29 and the multiple-tomographic-image constructing circuit 121.

The operation different from that of the first embodiment will be described below with reference to Fig. 19.

Image data of ultrasonic tomographic images required for constructing a three-dimensional image is recorded in the HDD 26 in advance in the same manner as the steps S11 to S16 according to the first embodiment.

When a user presses the DPR key 122 on the keyboard 5, the processing in Fig. 19 is started.

When the DPR key 122 is pressed, the cube data creating circuit 41 performs the same steps as the steps S21 and S22 of the first embodiment to create cube data CD (S71). The multiple-tomographic-image constructing circuit 121 extracts image data of a plane perpendicular to the x-axis and a plane perpendicular to the y-axis of the cube data CD shown in Fig. 6 in response to an instruction from the control circuit 29 (S72). The ultrasonic tomographic image including image data of the plane perpendicular to the x-axis and the ultrasonic tomographic image including image data of the plane perpendicular to the y-axis are called ultrasonic tomographic image P (131) and ultrasonic tomographic image Q (132), respectively, here. The positions of the planes of the ultrasonic tomographic image P (131) and ultrasonic tomographic image Q (132) are set at the center, that is, at x=L/2 and y=D/2, of the cube data shown in Fig. 6.

Next, the multiple-tomographic-image constructing circuit 121 superposes a crossing line of the plane P and the plane Q on the ultrasonic tomographic image P (131) (S73). The cut line on the ultrasonic tomographic image P (131) is called cut line P (133) here. The multiple-tomographic-image constructing circuit 121 superposes the crossing line of the plane P and the plane Q on the ultrasonic tomographic image Q (132) (S74). The cut line on the ultrasonic tomographic image Q (132) is called cut line Q (134) below.

At a step S75, the multiple-tomographic-image constructing circuit 121 outputs to the display circuit 28 multiple ultrasonic tomographic images having different directions, that is, image data having the aligned ultrasonic tomographic image P (131) and ultrasonic tomographic image Q (132). The display circuit 28 converts the image data of the multiple tomographic images to analog video signals, which can be displayed on the monitor 6 and outputs the analog video signals (S76). The monitor 6 displays multiple tomographic images having the colored bloodstream 135 shown in Fig. 20 (S77). In this way, the multiple-tomographic-image processing circuit 121 creates and displays on the monitor 6 multiple tomographic images having different directions from each other and superposing bloodstream images on tomographic images based on color data.

When a user moves the trackball 102, the movement of the trackball 102 is detected. Therefore, YES is determined at a step S78, and the processing goes to a step S79. If the trackball 102 is not moved, no processing is performed. At the step S79, the control circuit 29 outputs information on the direction and distance for the movement on the monitor screen 86 to the multiple-tomographic-image constructing circuit 121 as a tomographic position specifying signal based on the output of the trackball 102. Next, the multiple-tomographic-image constructing circuit 121 extracts the ultrasonic tomographic image P (131) perpendicular to the x-axis and the ultrasonic tomographic image Q (132) perpendicular to the y-axis again based on the tomographic position specifying signal included in the instruction from the control circuit 29 (S80). The positions of the planes of the ultrasonic tomographic image P (131) and ultrasonic tomographic image Q (132) are newly determined from the contents of the tomographic position specifying signal (S80). At a step S81, whether the user has pressed the DPR key 122 again is determined. If the user has pressed, YES is determined. Then, the processing ends in response to the instruction of the control circuit 29. Otherwise, the processing jumps to the step S73, and the processing is performed again.

Therefore, when the bloodstream 135 is checked on the ultrasonic tomographic image P, for example, and the cut line 133 is placed on the part by using the trackball 102, the ultrasonic tomographic image Q (132) corresponding to the position of the cut line 133 is displayed on the right side. Thus, the traveling of the bloodstream 135 can be observed from two directions. The other operation is the same as that of the first embodiment.

In this way, according to this embodiment, as shown in Fig. 20, the aligned multiple ultrasonic tomographic images P (131) and Q (132) having different directions are displayed on the monitor screen 86 at the same time as shown in Fig. 20. Thus, in a case of an esophageal varix, for example, the running of the bloodstream along the gullet can be displayed more easily and more clearly on the ultrasonic tomographic image P (131) or on the ultrasonic tomographic image Q (132) by slowly retracting an endoscope inserting portion, which has been inserted by a user to the gullet. Fig. 20 shows a state where the electronic radial scanning type ultrasonic endoscope 2 is retracted in the x-axis direction of the orthogonal coordinate system to which the receiving coil 21 is fixed. Here, the ultrasonic tomographic image P and the ultrasonic tomographic image Q correspond to the tomographic images perpendicular and parallel, respectively, to the endoscope inserting axis. According to this method, the traveling of the bloodstream, which is difficult to represent in a conventional ultrasonic endoscope, can be displayed by being colored by using color data. This is effective for diagnosis of an esophageal varix requiring the diagnosis of the state of the varix in the bloodstream and/or the traveling of the bloodstream bypass, for example. The other advantages are the same as those of the first embodiment.

In this way, ultrasonic tomographic images in multiple directions can be constructed easily from voxel data.

According to this embodiment, for displaying the tissue including a tumor by an ultrasonic three-dimensional image in the in-body-cavity ultrasonic diagnosis field, an ultrasonic diagnosis apparatus which can obtain good image data having no distortion by the easy operation can be realized.

Furthermore, according to this embodiment, in the in-body-cavity ultrasonic field, for displaying the bloodstream and the tissue including the bloodstream, an ultrasonic diagnosis apparatus which can obtain the bloodstream information using Doppler effect by the easy operation can be realized.

Next, a variation example will be described.

This embodiment describes the form of so-called DPR display for displaying two orthogonal ultrasonic tomographic images. However, multi-plane reconstruction (MPR) display may be adopted for displaying more ultrasonic tomographic images on the screen. The ultrasonic tomographic images do not have to be orthogonal in some cases and conditions.

Having described the preferred embodiments of the invention referring to the accompanying drawings, it should be understood that the present invention is not limited to those precise embodiments and various changes and modifications thereof could be made by one skilled in the art.

**Claims**

1. An ultrasonic diagnosis apparatus for obtaining ultrasonic echo signals of an object from a probe in a body cavity, comprising:

   an ultrasonic probe (2) having at predetermined positions of an inserting axis a plurality of ultrasonic transducers on the circumference about the inserting axis for performing ultrasonic scanning on a plane perpendicular to the inserting axis by sending and receiving ultrasound to and from the plurality of ultrasonic transducers;
   multiple-tomographic-image processing means (121) for creating multiple ultrasonic tomographic images having different directions from each other, based on ultrasonic tomographic image data from echo signals serially obtained by the ultrasonic scanning of the ultrasonic probe and
   detecting means (3) for detecting the position or orientation of a plane scanned by the ultrasonic probe (2), **characterized in that** the detecting means (3) are adapted to detect the position or orientation of a scanned plane remotely by means of an alternating magnetic field generated at the ultrasonic probe (2).

2. The ultrasonic diagnosis apparatus according to claim 1, **characterized in that** the multiple-tomographic-image processing means (121) creates the multiple ultrasonic tomographic images having different directions from each other, based on data of the position or orientation.

**3.** The ultrasonic diagnosis apparatus according to claim 1, further **characterized by** comprising:

Doppler signal processing means (34) for creating color data of the bloodstream by using Doppler effect of echo signals from the ultrasonic probe,
**characterized in that** the multiple-tomographic image processing means (121) creates the multiple ultrasonic tomographic images having different directions from each other and superposed by an image of the bloodstream based on the color data thereon.

**4.** The ultrasonic diagnosis apparatus according to claim 2, further **characterized by** comprising:

Doppler signal processing means (34) for creating color data of the bloodstream by using Doppler effect of echo signals from the ultrasonic probe,
**characterized in that** the multiple-tomographic image processing means (121) creates the multiple ultrasonic tomographic images having different directions from each other and superposed by an image of the bloodstream based on the color data thereon.

**5.** The ultrasonic diagnosis apparatus according to claim 1, **characterized in that** the ultrasonic probe is an ultrasonic endoscope having an optical observation window at the distal end.

**Patentansprüche**

**1.** Ultraschalldiagnosegerät zum Erhalten von Ultraschall-Echosignalen eines Objekts von einer Sonde in einer Körperhöhle, aufweisend:

einer Ultraschallsonde (2), die an vorbestimmten Stellen einer Einführachse eine Mehrzahl von Ultraschallwandlern am Umfang um die Einführachse aufweist, um einen Ultraschall-Abtastvorgang in einer zu der Einführachse rechtwinkligen Ebene durch Aussenden von Ultraschall zu der Vielzahl von Ultraschallwandlern sowie Empfangen von Ultraschall von der Vielzahl von Ultraschallwandlern auszuführen;
Mehrfachschichtbild-Verarbeitungsmitteln (121) zum Erzeugen von mehreren, in verschiedenen Richtungen zueinander ausgerichteten Ultraschall-Schichtbildern basierend auf von aus Echosignalen erhaltenen Ultraschall-Schichtbilddaten,
wobei die Echosignale seriell durch den Ultraschall-Abtastvorgang der Ultraschallsonde erhalten werden, und
Detektionsmitteln (3) zum Detektieren der Position oder Orientierung einer Ebene, die von der Ultraschallsonde (2) abgetastet wird,
**dadurch gekennzeichnet, dass** die Detektionsmittel (3) ausgebildet sind, um die Position oder Orientierung einer abgetasteten Ebene mittels eines an der Ultraschallsonde (2) erzeugten Magnetwechselfelds aus der Ferne zu erfassen.

**2.** Ultraschalldiagnosegerät nach Anspruch 1,
**dadurch gekennzeichnet, dass** die Mehrfachschichtbild-Verarbeitungsmittel (121) die mehreren, in verschiedenen Richtungen zueinander ausgerichteten Ultraschall-Schichtbilder basierend auf Positionsdaten oder Orientierungsdaten erzeugen.

**3.** Ultraschalldiagnosegerät nach Anspruch 1, ferner **gekennzeichnet durch**:

Dopplersignal-Verarbeitungsmittel (34) zum Erzeugen von Farbdaten des Blutstroms **durch** Verwendung des Doppler-Effekts von Echosignalen der Ultraschallsonde,
**dadurch gekennzeichnet, dass** die Mehrfachschichtbild-Verarbeitungsmittel (121) die mehreren Ultraschall-Schichtbilder erzeugen, die in verschiedenen Richtungen zueinander ausgerichtet und basierend auf den Farbdaten von einem Bild des Blutstroms überlagert sind.

**4.** Ultraschalldiagnosegerät nach Anspruch 2, ferner **gekennzeichnet durch**:

Dopplersignal-Verarbeitungsmittel (34) zum Erzeugen von Farbdaten des Blutstroms **durch** Verwendung des Doppler-Effekts von Echosignalen der Ultraschallsonde,
**dadurch gekennzeichnet, dass** die Mehrfachschichtbild-Verarbeitungsmittel (121) die mehreren Ultraschall-Schichtbilder erzeugen, die in verschiedenen Richtungen zueinander ausgerichtet und basierend auf den Farbdaten von einem Bild des Blutstroms überlagert sind.

**5.** Ultraschalldiagnosegerät nach Anspruch 1, **dadurch gekennzeichnet, dass** es sich bei der Ultraschallsonde um ein Ultraschallendoskop handelt, das an dem distalen Ende ein optisches Beobachtungsfenster aufweist.

## Revendications

**1.** Appareil de diagnostic par ultrasons pour obtenir des signaux d'écho ultrasoniques d'un objet à partir d'une sonde dans une cavité d'un corps, comprenant :

une sonde ultrasonique (2) ayant en des positions prédéterminées d'un axe d'insertion une pluralité de transducteurs ultrasoniques sur la circonférence autour de l'axe d'insertion pour effectuer un balayage ultrasonique sur un plan perpendiculaire à l'axe d'insertion par envoi et réception d'ultrasons vers et de la pluralité de transducteurs ultrasoniques ;
un moyen de traitement d'images tomographiques multiples (121) pour créer des images tomographiques ultrasoniques multiples ayant différentes directions les unes par rapport aux autres, sur le base de données d'images tomographiques ultrasoniques provenant des signaux d'écho obtenus en série par le balayage ultrasonique de la sonde ultrasonique, et
un moyen de détection (3) pour détecter la position ou l'orientation d'un plan balayé par la sonde ultrasonique (2), **caractérisé en ce que** le moyen de détection (3) est adapté à détecter la position ou l'orientation d'un plan balayé à distance au moyen d'un champ magnétique alternatif généré au niveau de la sonde ultrasonique (2).

**2.** Appareil de diagnostic par ultrasons selon la revendication 1,
**caractérisé en ce que** le moyen de traitement d'images tomographiques multiples (121) crée les images tomographiques ultrasoniques multiples ayant différentes directions les unes par rapport aux autres, sur la base de données de la position ou de l'orientation.

**3.** Appareil de diagnostic par ultrasons selon la revendication 1, **caractérisé en outre en ce que** comprenant :

un moyen de traitement de signaux Doppler (34) pour créer des données en couleur du flux sanguin en utilisant l'effet Doppler de signaux d'écho provenant de la sonde ultrasonique,
**caractérisé en ce que** le moyen de traitement d'images tomographiques multiples (121) crée les images tomographiques ultrasoniques multiples ayant différentes directions les unes par rapport aux autres et superposées par une image du flux sanguin sur la base des données de couleur sur celui-ci.

**4.** Appareil de diagnostic par ultrasons selon la revendication 2, **caractérisé en outre en ce que** comprenant :

un moyen de traitement de signaux Doppler (34) pour créer des données en couleur du flux sanguin en utilisant l'effet Doppler de signaux d'écho provenant de la sonde ultrasonique,
**caractérisé en ce que** le moyen de traitement d'images tomographiques multiples (121) crée les images tomographiques ultrasoniques multiples ayant différentes directions les unes par rapport aux autres et superposées par une image du flux sanguin sur la base des données de couleur sur celui-ci.

**5.** Appareil de diagnostic par ultrasons selon la revendication 1, **caractérisé en ce que** la sonde ultrasonique est un endoscope ultrasonique ayant une fenêtre d'observation optique à l'extrémité distale.

# FIG.1

# FIG.2

ULTRASONIC
BEAM

RS

51 52 56

ROTATION

15

54 53

55

COIL EXCITING
SIGNAL

SEND DRIVING
VOLTAGE ULTRASONIC
ECHO SIGNAL

TO ULTRASONIC
PROCESSING UNIT

14 13

# FIG.3

SEND SWITCHING CIRCUIT

63

SEND DRIVING VOLTAGE

SEND DELAY CIRCUIT

62

SEND DRIVING VOLTAGE

SEND DRIVING VOLTAGE GENERATING CIRCUIT

61

SWITCHING CONTROL SIGNAL

RECEIVE SWITCHING CIRCUIT

64

ULTRASONIC ECHO SIGNAL

65

ULTRASONIC ECHO SIGNAL

RECEIVE DELAY CIRCUIT

66

ULTRASONIC ECHO SIGNAL

ADDING CIRCUIT

67

RECEIVE BEAM SIGNAL

68

SEND/RECEIVE CONTROLLER

ROTATIONAL ANGLE DATA

TO DSC(D)    TO DSC(B)

TO DOPPLER PROCESSING CIRCUIT

TO B-MODE PROCESSING CIRCUIT

31

56

# FIG.4

START

ULTRASONIC TRANSDUCER ARRAY
PERFORMS RADIAL SCANNING — S11

POSITION DETECTING
UNIT CREATES
POSITION/ORIENTATION
DATA OF SCANNED PLANE — S12

ULTRASONIC SIGNAL
PROCESSING CIRCUIT
CREATES IMAGE DATA
SUPERPOSING COLOR DATA — S13

SYNCHRONOUS WRITING CIRCUIT
RECORDS POSITION/ORIENTATION
DATA AND IMAGE DATA, WHICH
ARE ASSOCIATED SYNCHRONOUSLY — S14

INPUT TO
SCANNING CONTROL
KEY AGAIN
? — S15

NO

YES

ULTRASONIC TRANSDUCER ARRAY
STOPS RADIAL SCANNING — S16

END

21

# FIG.5

START

CUBE DATA CREATING CIRCUIT READS OUT IMAGE DATA AND POSITION/ORIENTATION DATA /S21

CUBE DATA CREATING CIRCUIT CREATES CUBE DATA /S22

COLOR PART EXTRACTING CIRCUIT EXTRACTS A BLOODSTREAM PART FROM CUBE DATA /S23

THREE-DIMENSIONAL IMAGE CONSTRUCTING CIRCUIT CONSTRUCTS A THREE-DIMENSIONAL IMAGE FROM THE BLOODSTREAM PART /S24

DISPLAY CIRCUIT CONVERTS IMAGE DATA OF THE THREE-DIMENSIONAL IMAGE TO ANALOG VIDEO SIGNAL AND OUTPUTS THE ANALOG VIDEO SIGNAL /S25

END

# FIG.6

# FIG.7

EP 1 669 030 B1

# FIG.8

# FIG.9

# FIG.10

EP 1 669 030 B1

# FIG.11

START

S31 — PROCESSING FROM S11 TO S15

S32 — PROCESSING FROM S21 TO S23

S33 — SWITCHING CONTROL KEY PRESSED ? — NO

YES

S34 — TURN SWITCH TO TERMINAL B

S35 — SCANNING CONTROL KEY PRESSED ? — NO

YES

S36 — ULTRASONIC TRANSDUCER ARRAY PERFORMS RADIAL SCANNING

S37 — TOMOGRAPHIC SUPERPOSING CIRCUIT WRITES SCHEMATIC DIAGRAM OF SCANNED PLANE IN CUBE DATA SPACE

S38 — THREE-DIMENSIONAL IMAGE CONSTRUCTING CIRCUIT CONSTRUCTS GUIDE IMAGE

S39 — MIXER CIRCUIT MIXES GUIDE IMAGE AND ULTRASONIC TOMOGRAPHIC IMAGE

S40 — DISPLAY CIRCUIT CONVERTS MIXED IMAGE DATA TO ANALOG VIDEO SIGNAL AND OUTPUTS ANALOG VIDEO SIGNAL

S41 — SCANNING CONTROL KEY PRESSED AGAIN? — NO

YES

S42 — ULTRASONIC TRANSDUCER ARRAY STOPS RADIAL SCANNING

S43 — TURN SWITCH TO TERMINAL A

END

# FIG.12

# FIG.13

# FIG.14

EP 1 669 030 B1

# FIG.15

EP 1 669 030 B1

# FIG.16

```
                    ( START )
                         │
   S51 ──┐   ┌────────────────────────────────┐
         └──>│    PROCESSING FROM S21 TO S23   │
             └────────────────────────────────┘
                         │
   S52 ──┐   ┌────────────────────────────────┐
         └──>│ TOMOGRAPHIC POSITION SUPERPOSING│
             │  CIRCUIT SUPERPOSES TOMOGRAPHIC │
             │    POSITION SPECIFYING CURSOR   │
             └────────────────────────────────┘
                         │
   S53 ──┐   ┌────────────────────────────────┐
         └──>│ THREE-DIMENSIONAL CONSTRUCTING  │
             │   CIRCUIT CONSTRUCTS GUIDE IMAGE│
             └────────────────────────────────┘
                         │
   S54 ──┐   ┌────────────────────────────────┐
         └──<│       DISPLAY (GUIDE IMAGE)     │
             └────────────────────────────────┘
                         │
   S55 ──┐        ◇─────────────────◇         NO
         └──<│   TRACKBALL MOVED?    │──────────>
              ◇─────────────────◇
                      │ YES
   S56 ──┐   ┌────────────────────────────────┐
         └──>│ CONTROL CIRCUIT OUTPUTS TOMOGRAPHIC│
             │    POSITION SPECIFYING SIGNAL   │
             └────────────────────────────────┘
                         │
   S57 ──┐   ┌────────────────────────────────┐
         └──>│  READING CIRCUIT READ OUT POSITION/│
             │  ORIENTATION DATA AND IMAGE DATA │
             └────────────────────────────────┘
                         │
   S58 ──┐   ┌────────────────────────────────┐
         └──>│ TOMOGRAPHIC POSITION SUPERPOSING│
             │ CIRCUIT SUPERPOSES NEW TOMOGRAPHIC│
             │    POSITION SPECIFYING CURSOR   │
             └────────────────────────────────┘
                         │
   S59 ──┐   ┌────────────────────────────────┐
         └──>│ THREE-DIMENSIONAL CONSTRUCTING  │
             │   CIRCUIT CONSTRUCTS GUIDE IMAGE│
             └────────────────────────────────┘
                         │
   S60 ──┐   ┌────────────────────────────────┐
         └──>│ MIXER CIRCUIT MIXES GUIDE IMAGE AND│
             │   ULTRASONIC TOMOGRAPHIC IMAGE  │
             └────────────────────────────────┘
                         │
   S61 ──┐   ┌────────────────────────────────┐
         └──<│      DISPLAY (GUIDE IMAGE+      │
             │  ULTRASONIC TOMOGRAPHIC IMAGE)  │
             └────────────────────────────────┘
                         │
   S62 ──┐        ◇─────────────────◇         NO
         └──<│  3D KEY PRESSED AGAIN │──────────>
              ◇─────────────────◇
                      │ YES
                    ( END )
```

# FIG.17

# FIG.18

EP 1 669 030 B1

# FIG.19

START

S71 — PROCESSING FROM S21 TO S22

S72 — MULTIPLE-TOMOGRAPHY-IMAGE CONSTRUCTING CIRCUIT EXTRACTS IMAGE DATA OF ULTRASONIC TOMOGRAPHIC IMAGES P AND Q

S73 — MULTIPLE-TOMOGRAPHY-IMAGE CONSTRUCTING CIRCUIT SUPERPOSES CUT LINE P

S74 — MULTIPLE-TOMOGRAPHY-IMAGE CONSTRUCTING CIRCUIT SUPERPOSES CUT LINE Q

S75 — MULTIPLE-TOMOGRAPHY-IMAGE CONSTRUCTING CIRCUIT OUTPUTS ULTRASONIC TOMOGRAPHIC IMAGES P AND Q IN A LINE TO DISPLAY CIRCUIT

S76 — DISPLAY CIRCUIT CONVERTS IMAGE DATA OF MULTIPLE-TOMOGRAPHY IMAGES TO ANALOG VIDEO SIGNALS AND OUTPUT THE ANALOG VIDEO SIGNALS

S77 — DISPLAY (MULTIPLE-TOMOGRAPHY IMAGE)

S78 — TRACKBALL MOVED? — NO

YES

S79 — CONTROL CIRCUIT OUTPUTS TOMOGRAPHIC POSITION SPECIFYING SIGNAL

S80 — MULTIPLE-TOMOGRAPHY IMAGE CONSTRUCTING CIRCUIT EXTRACTS IMAGE DATA OF NEW ULTRASONIC TOMOGRAPHIC IMAGES P AND Q

S81 — DPR KEY PRESSED AGAIN? — NO

YES

END

# FIG.20

EP 1 669 030 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6254097 A
- JP 2000242766 A
- JP 2001161693 A
- JP 6261900 A
- JP 11113913 A
- US 5876345 A
- US 6248074 B1